(19) **Europäisches Patentamt / European Patent Office / Office européen des brevets**

(11) **EP 3 325 605 B1**

(12) **FASCICULE DE BREVET EUROPEEN**

(45) Date de publication et mention de la délivrance du brevet:
**06.09.2023 Bulletin 2023/36**

(21) Numéro de dépôt: **16750384.6**

(22) Date de dépôt: **13.07.2016**

(51) Classification Internationale des Brevets (IPC):
**C12N 1/12** *(2006.01)*     **C12P 21/00** *(2006.01)*

(52) Classification Coopérative des Brevets (CPC):
**C12N 1/12; A23K 10/16; A23K 20/00; A23K 20/147; A23K 50/75; A23L 17/60; A23L 33/135; A23L 33/195; A61K 8/9728; A61K 36/06; A61P 3/02; A61P 17/00; A61P 17/16; A61Q 19/00;** A61K 2800/10

(86) Numéro de dépôt international:
**PCT/EP2016/066590**

(87) Numéro de publication internationale:
**WO 2017/012931 (26.01.2017 Gazette 2017/04)**

(54) **BIOMASSE DE THRAUSTOCHYTRIDES RICHE EN PROTEINES, PROCEDE DE CULTURE ET UTILISATIONS**

PROTEINREICHE BIOMASSE VON THRAUSTOCHYTRIDEN, KULTIVIERUNGSVERFAHREN UND VERWENDUNGEN

PROTEIN-RICH BIOMASS OF THRAUSTOCHYTRIDS, CULTURING METHOD, AND USES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.07.2015 FR 1556791**

(43) Date de publication de la demande:
**30.05.2018 Bulletin 2018/22**

(83) **Déclaration conformément à la règle 32(1) CBE (solution de l'expert)**

(73) Titulaires:
- **Fermentalg**
  **33500 Libourne (FR)**
- **Adisseo France S.A.S.**
  **92160 Antony (FR)**

(72) Inventeurs:
- **CAGNAC, Olivier**
  **33500 Libourne (FR)**
- **ROLS, Cyril**
  **13004 Marseille (FR)**
- **PAGLIARDINI, Julien**
  **33000 Bordeaux (FR)**
- **CALLEJA, Pierre**
  **33450 Saint Sulpice et Cameyrac (FR)**
- **GADY, Cécile**
  **03100 Montluçon (FR)**
- **VANDEPLAS, Sabrina**
  **03420 Terjat (FR)**

(74) Mandataire: **Icosa**
  **83 avenue Denfert-Rochereau**
  **75014 Paris (FR)**

(56) Documents cités:
**WO-A1-2012/175027     WO-A1-2016/120558 WO-A2-2015/004402**

- **SUN LINA ET AL: "Differential effects of nutrient limitations on biochemical constituents and docosahexaenoic acid production ofSchizochytriumsp", BIORESOURCE TECHNOLOGY, vol. 159, 4 mars 2014 (2014-03-04), pages 199-206, XP028646306, ISSN: 0960-8524, DOI: 10.1016/J.BIORTECH.2014.02.106**

- YAMASAKI T ET AL: "Utilization of Shochu distillery wastewater for production of polyunsaturated fatty acids and xanthophylls using thraustochytrid", JOURNAL OF BIOSCIENCE AND BIOENGINEERING, ELSEVIER, AMSTERDAM, NL, vol. 102, no. 4, 1 octobre 2006 (2006-10-01), pages 323-327, XP028042220, ISSN: 1389-1723, DOI: 10.1263/JBB.102.323 [extrait le 2006-10-01]
- LIU YING ET AL: "Culturable diversity and biochemical features of thraustochytrids from coastal waters of Southern China", APPLIED MICROBIOLOGY AND BIOTECHNOLOGY, SPRINGER, DE, vol. 98, no. 7, 24 novembre 2013 (2013-11-24), pages 3241-3255, XP035329079, ISSN: 0175-7598, DOI: 10.1007/S00253-013-5391-Y [extrait le 2013-11-24]

**Description**

**DOMAINE DE L'INVENTION**

L'invention se place dans le domaine des cultures de microalgues particulièrement des Thraustochytrides. Elle a pour objets une biomasse de Thraustochytrides riche en protéines, son procédé d'obtention et ses utilisations dans l'alimentation.

**ETAT DE LA TECHNIQUE**

On connaît plusieurs sources de protéines végétales pour leur utilisation dans l'alimentation humaine ou animale, directement ou comme compléments alimentaires, pour apporter aux animaux et humains des acides aminés nécessaires à leur métabolisme. Ces sources de protéines sont entendues comme des sources d'acides aminés disponibles pour les animaux ou humains une fois les aliments ingérés.

La plus connue des sources de protéines végétales employée dans l'alimentation animale est le soja, généralement employé sous forme de tourteaux, résidu solide restant après extraction de l'huile. Toutefois, l'emploi de tourteaux de soja présente plusieurs inconvénients associés à leur origine. Les tourteaux sont généralement importés de pays qui pratiquent une culture intensive du soja au détriment d'autres végétaux source de biodiversité. En outre, de nombreux pays favorisent la culture de variétés de soja génétiquement modifiées (OGM), que l'on retrouve mélangées aux sojas non OGM dans les tourteaux, ce qui ne permet pas de répondre à une demande croissante de produits alimentaires d'origine végétale sans OGM.

Des aliments pour animaux à base de biomasse constituée de microorganismes photosynthétiques génétiquement modifiés sont connus de WO 2010/051489. L'enzyme recombinante produite par le microorganisme OGM a pour effet de dégrader ladite biomasse pour la rendre compatible avec l'alimentation des animaux.

On connaît d'autres sources de protéines végétales, notamment la spiruline ou la chlorelle, employées comme compléments alimentaires chez l'homme.

La spiruline, comme la chlorelle, présentent toutefois l'inconvénient d'une faible productivité qui ne permet pas le passage à une culture en fermenteur à rendement élevé. Si leur culture permet de répondre à une demande locale et limitée en compléments alimentaires traditionnels, elle ne permet par de répondre à un objectif de production industrielle plus large, économiquement viable, d'une source de protéines alimentaires dont les qualités lui permettront de remplacer les sources usuelles comme le soja dans l'alimentation des animaux et de l'homme.

A l'inverse, les protistes connus pour répondre à une capacité de production industrielle en fermenteurs, sont employés depuis longtemps pour la production de matières grasses riches en acides gras polyinsaturés comme le DHA ou l'EPA, par exemple WO 97/37032, WO 2015/004402 ou WO 2012/175027. Or, les biomasses obtenues, y compris après extraction des matières grasses, ne contiennent pas de teneurs suffisantes en protéines pour permettre leur usage comme source de protéines dans l'alimentation, pour le moins sans étapes additionnelles d'enrichissement en protéines économiquement coûteuses. En outre, les méthodes employées pour l'extraction des huiles peuvent parfois contaminer la biomasse restante, notamment avec des solvants organiques qui la rendent inapte à une consommation alimentaire.

L'un des buts que vise l'invention est de fournir une nouvelle source de protéines pour l'alimentation animale ou humaine répondant à un objectif de production industrielle large, économiquement viable, et dont les qualités lui permettront de remplacer les sources usuelles comme le soja.

L'invention montre que dans certaines conditions de culture les Thraustochytrides, connues pour leur utilisation dans la production d'huiles à fortes teneurs en acides gras polyinsaturés (DHA, EPA notamment) sont des microorganismes aptes à produire une grande quantité de protéines ce qui peut en faire une source de protéines alimentaires similaires au soja, en particulier pour l'alimentation animale.

**EXPOSE DE L'INVENTION**

Ainsi l'invention a pour objet premier une biomasse de Thraustochytrides non génétiquement modifiés choisi parmi les genres *Aurantiochytrium* et *Schyzochytrium,* dont une quantité substantielle majoritaire de Thraustochytrides n'est pas dégradée, qui comprend, en poids par rapport au poids de la matière sèche, au moins 35% de protéines, préférentiellement au moins 45 % de protéines, pouvant aller jusqu'à plus de 60 % de protéines, voire plus de 75 % de protéines, en particulier de 45 à 75 % de protéines.

Les pourcentages en poids en protéines peuvent être exprimés tant par rapport aux protéines elles-mêmes que par rapport aux acides aminés contenus dans lesdites protéines.

Ladite biomasse comprend également, en poids par rapport au poids de la matière sèche, moins de 20% de matière grasse, préférentiellement moins de 1 0% de matière grasse, encore plus préférentiellement moins de 7% de matière grasse.

Selon l'invention ladite biomasse est une biomasse de Thraustochytrides non génétiquement modifiés choisi parmi les genres *Aurantiochytrium* et *Schyzochytrium,* dont une quantité substantielle majoritaire de Thraustochytrides n'est pas dégradée, qui comprend, en poids par rapport au poids de la matière sèche, au moins 35 %, préférentiellement au moins 45 %, très préférentiellement de 45% à 60% de protéines et, toujours en poids par rapport au poids de la matière sèche, moins de 20%, préférentiellement moins de 10% de matière grasse, encore plus préférentiellement moins de 7% de matière grasse.

L'invention concerne également un procédé de production d'une biomasse telle que définie ci-dessus et ci-après, caractérisé en ce qu'il comprend :

a. Une première étape de culture des Thraustochytrides dans un milieu de culture défini comprenant une source de carbone, une source d'azote, une source de phosphore et des sels, ledit milieu défini ne comportant pas de matières organiques riches ou complexes, et ladite première étape a) étant divisée en 2 sous-étapes, une première sous-étape

a1) de croissance dans le milieu de culture approprié jusqu'à obtenir des teneurs en source de carbone dans le milieu inférieur à 20 g/L suivie d'une deuxième sous-étape

a2) de production dans laquelle on ajoute au milieu de culture, simultanément ou successivement, une ou plusieurs solution(s) d'enrichissement en source de carbone pour maintenir la teneur en source de carbone entre 0 et 50 g/L, en source d'azote pour maintenir la teneur en azote entre 0,5 et 5 g/L et/ou en source de phosphore pour maintenir la teneur en phosphore entre 0,5 et 5 g/L de manière à maintenir dans le milieu de culture des teneurs en azote et en phosphore non limitantes pour la croissance jusqu'à l'obtention d'une densité de culture d'au moins 40 g/L en matière sèche ;

b. une deuxième étape de récupération de la biomasse obtenue à la première étape par séparation de ladite biomasse du milieu de culture (récolte); et, le cas échéant

c. une troisième étape de séchage de la biomasse récupérée à la deuxième étape.

L'invention concerne aussi l'utilisation d'une biomasse telle que ci-dessus ou ci-après, dans les domaines cosmétiques et alimentaires humain ou animal, et notamment un aliment comprenant une telle biomasse.

L'invention concerne également la biomasse selon l'invention pour son utilisation en thérapie.

Elle concerne également des compositions cosmétique ou pharmaceutique pour l'homme ou les animaux et des aliments, ou compositions alimentaires pour l'homme ou les animaux, qui comprennent une biomasse selon l'invention.

**DESCRIPTION DETAILLEE DE L'INVENTION**

On entend avantageusement par "biomasse" selon l'invention un ensemble de cellules de Thraustochytrides produites par la culture desdits protistes, et présentant les teneurs en protéines et, éventuellement en acides gras décrites dans le présent texte, cellules qui peuvent avoir conservé ou non leur intégrité physique.

On comprend donc que ladite biomasse peut comprendre une quantité de cellules de Thraustochytrides dégradées allant de 0% à 100%. Par "dégradée" on entend que lesdites cellules de Thraustochytrides peuvent avoir vu leur structure et/ou leur composition modifiées. Par exemple elles peuvent avoir subi une étape de séchage ou alors une étape de récolte de leur huile, l'important étant que la biomasse comprenant ces cellules présente les teneurs en protéines et, éventuellement en acides gras, décrites dans le présent texte.

Selon un mode préféré de réalisation de l'invention, la biomasse n'a pas subi de traitements qui modifient sa composition en acides aminés au cours ou après récolte. C'est à dire que les traitements que subit cette biomasse après récolte ne viennent pas en altérer la composition en acides aminés. En particulier, la biomasse ne subit pas d'étape d'enrichissement en protéines et/ou en acides aminés. C'est à dire les protéines, peptides et acides aminés contenus dans la biomasse selon l'invention proviennent de la seule culture des Thraustochytrides. Il convient de noter que des protéines ou acides aminés non produits par les Thraustochytides sont susceptibles d'être présents dans le milieu de culture, notamment en cas de préculture sur un milieu comprenant un extrait de levure. Les quantités résiduelles de ces protéines susceptibles d'être présentes dans la biomasse, le cas échéant, le seront à l'état de traces non détectables, inclues dans la définition d'une biomasse qui ne subit pas d'enrichissement en protéines et/ou en acides aminés.

Selon un mode plus préféré de l'invention, la biomasse n'a pas subi de traitements qui modifient sa composition en acides aminés et en matière grasse. C'est à dire que les traitements que subit cette biomasse après récolte ne viennent pas en altérer la composition en acides aminés et en matières grasses. En particulier, la composition relative des acides aminés par rapport à la matière grasse reste substantiellement constante.

Il a été observé dans certains cas que les Thraustochytrides non dégradées dans la biomasse selon l'invention présentent de meilleures propriétés de conservation et de digestibilité que des Thraustochytrides dégradées. Une des formes préférées de l'invention est une biomasse comprenant une quantité substantiellement majoritaire de Thraustochytrides qui ne sont pas dégradées.

Par dégradé on entend selon l'invention des Thraustochytrides dont l'intégrité structurelle et/ou chimique a pu être altérée comme par exemple des Thraustochytrides lysées, résultant par exemple d'un procédé d'homogénéisation.

Il n'en demeure pas moins évident qu'une fois produite ladite biomasse pourra être utilisée brute, séchée ou non, ou alors subir tout traitement nécessaire à son utilisation, notamment homogénéisée.

Selon l'invention ladite biomasse peut présenter, en poids par rapport au poids de la matière sèche, un taux d'humidité de 1% à 95%.

Préférentiellement selon une première variante de l'invention ladite biomasse peut présenter, en poids par rapport au poids de la matière sèche, un taux d'humidité de 70 à 90 %, préférentiellement 80 à 85 %.

Préférentiellement encore selon une deuxième variante de l'invention ladite biomasse peut présenter, en poids par rapport au poids de la matière sèche, un taux d'humidité de 1 à 10%, préférentiellement de 2 à 7 %.

Selon l'invention lesdits Thraustochytrides peuvent être de l'ordre des Thraustochytriales, préférentiellement de la sous-classe des Thraustochytriaceae, encore plus préférentiellement d'un genre pouvant être choisi dans le groupe comprenant les genres *Aurantiochytrium, Aplanochytrium, Botryochytrium, Japonochytrium, Oblongichytrium, Parietichytrium, Schizochytrium, Sicyoidochytrium, Thraustochytrium* et *Ulkenia.*

Les Thraustochytrides sont très préférentiellement des microorganismes non génétiquement modifiés.

Avantageusement lesdits Thraustochytrides peuvent être choisis parmi les espèces *Aplanochytrium kerguelense ; Aplanochytrium minuta ; Aplanochytrium stocchinoi ; Aplanochytrium sp.* PR24-1 *; Aurantiochytrium limacinum ; Aurantiochytrium limacinum* AB022107 *; Aurantiochytrium limacinum* HM042909 *; Aurantiochytrium limacinum* JN986842 *; Aurantiochytrium limacinum* SL1101 JN986842 *; Aurantiochytrium mangrovei ; Aurantiochytrium mangrovei* DQ323157 *; Aurantiochytrium mangrovei* DQ356659 *; Aurantiochytrium mangrovei* DQ367049 *; Aurantiochytrium mangrovei* CCAP 4062/2 *; Aurantiochytrium mangrovei* CCAP 4062/3 *; Aurantiochytrium mangrovei* CCAP 4062/4 *; Aurantiochytrium mangrovei* CCAP 4062/5 *; Aurantiochytrium mangrovei* CCAP 4062/6 *; Aurantiochytrium mangrovei* CCAP 4062/1 *; Aurantiochytrium sp.* AB052555 *; Aurantiochytrium sp.* AB073308 *; Aurantiochytrium sp.* ATCC PRA276 DQ836628 *; Aurantiochytrium sp.* BL10 FJ821477 *; Aurantiochytrium sp.* LY 2012 PKU Mn5 JX847361 *; Aurantiochytrium sp.* LY2012 JX847370 *; Aurantiochytrium sp.* N1-27 *; Aurantiochytrium sp.* SD116 *; Aurantiochytrium sp.* SEK209 AB290574 *; Aurantiochytrium sp.* SEK217 AB290572 *; Aurantiochytrium sp.* SEK 218 AB290573 *; Aurantiochytrium sp.* 18W-13a *; Botryochytrium radiatum ; Botryochytrium radiatum* Raghukumar 16 *; Botryochytrium radiatum* SEK353 *; Botryochytrium sp. ; Botryochytrium sp.* BUTRBC 143 *; Botryochytrium sp.* Raghukumar 29 *; Oblongichytrium minutum ; Oblongichytrium multirudimentalis ; Oblongichytrium sp. ; Oblongichytrium sp.* SEK347 *; Parieticytrium sarkarianum ; Parieticytrium sarkarianum* SEK351 *; Parieticytrium sarkarianum* SEK364 *; Parieticytrium sp. ; Parieticytrium sp.* F3-1 *; Parieticytrium sp.* H1-14 *; Parieticytrium sp.* NBRC102984 *; Phytophthora infestans ; Schizochytrium aggregatum* DQ323159 *; Schizochytrium aggregatum* DQ356661 *; Schizochytrium aggregatum ; Schizochytrium limacinum ; Schizochytrium limacinum* OUC166 HM042907 *; Schizochytrium mangrovei ; Schizochytrium mangrovei* FB1 *; Schizochytrium mangrovei* FB3 *; Schizochytrium mangrovei* FB5 *; Schizochytrium minutum ; Schizochytrium sp.* ATCC20888 DQ367050 *; Schizochytrium sp.* KGS2 KC297137 *; Schizochytrium sp.* SKA10 JQ248009 *; Schizochytrium sp.* ATCC 20111 *; Schizochytrium sp.* ATCC 20888 *; Schizochytrium sp.* ATCC 20111 DQ323158* *; Schizochytrium sp.* ATCC 20888 DQ356660 *; Schizochytrium sp.* ATCC 20889 *; Schizochytrium sp.* ATCC 26185 *; Schizochytrium sp.* BR2.1.2 *; Schizochytrium sp.* BUCAAA 032 *; Schizochytrium sp.* BUCAAA 093 *; Schizochytrium sp.* BUCACD 152; *Schizochytrium sp.* BUCARA 021 *; Schizochytrium sp.* BUCHAO 113 *; Schizochytrium sp.* BURABQ 133 *; Schizochytrium sp.* BURARM 801 *; Schizochytrium sp.* BURARM 802 *; Schizochytrium sp.* CCAP 4087/3 *; Schizochytrium sp.* CCAP 4087/1 *; Schizochytrium sp.* CCAP 4087/4 *; Schizochytrium sp.* CCAP 4087/5 *;Schizochytrium sp.* FJU-512 *; Schizochytrium sp.* KH105 *; Schizochytrium sp.* KK17-3 *; Schizochytrium sp.* KR-5 *; Schizochytrium sp.* PJ10.4 *; Schizochytrium sp.* SEK 210 *; Schizochytrium sp.* SEK 345 *; Schizochytrium sp.* SEK 346 *; Schizochytrium sp.* SR21 *; Schizochytrium sp.* TIO01 *; Sicyoidochytrium minutum* SEK354 *; Sicyoidochytrium minutum* NBRC *102975Sicyoidochytrium minutum* NBRC 102979 *; Thraustochytriidae sp.* BURABG162 DQ100295 *; Thraustochytriidae sp.* CG9 *; Thraustochytriidae sp.* LY2012 JX847378 *; Thraustochytriidae sp.* MBIC11093 AB183664 *; Thraustochytriidae sp.* NIOS1 AY705769 *; Thraustochytriidae sp.* #32 DQ323161 *; Thraustochytriidae sp.* #32 DQ356663 *; Thraustochytriidae sp.* RT49 DQ323167 *; Thraustochytriidae sp.* RT49 DQ356669 *; Thraustochytriidae sp.* RT49 *; Thraustochytriidae sp.* Thel2 DQ323162 *; Thraustochytriidae* sp. Thel2 *; Thraustochytrium aggregatum ; Thraustochytrium aggregatum* DQ356662 *; Thraustochytrium aureum ; Thraustochytrium aureum* DQ356666 *; Thraustochytrium gaertnerium ; Thraustochytrium kinnei ; Thraustochytrium kinnei* DQ323165 *; Thraustochytrium motivum ; Thraustochytrium multirudimentale ; Thraustochytrium pachydermum ; Thraustochytrium roseum ; Thraustochytrium sp.* 13A4.1 *; Thraustochytrium* sp. ATCC 26185 *; Thraustochytrium sp.* BL13 *; Thraustochytrium sp.* BL14 *; Thraustochytrium sp.* BL2 *; Thraustochytrium sp.* BL3 *; Thraustochytrium sp.* BL4 *; Thraustochytrium sp.* BL5 *; Thraustochytrium sp.* BL6 *; Thraustochytrium sp.* BL7 *; Thraustochytrium sp.* BL8 *; Thraustochytrium sp.* BL9 *; Thraustochytrium sp.* BP3.2.2 *; Thraustochytrium sp.* BP3.3.3 *; Thraustochytrium sp.*

caudivorum ; *Thraustochytrium sp.* CHN-1 ; *Thraustochytrium sp.* FJN-10 ; *Thraustochytrium sp.* HK1 ; *Thraustochytrium sp.* HK10 ; *Thraustochytrium sp.* HK5 ; *Thraustochytrium sp.* HK8 ; *Thraustochytrium sp.* HK8a ; *Thraustochytrium sp.* KK17-3 ; *Thraustochytrium sp.* KL1 ; *Thraustochytrium sp.* KL2 ; *Thraustochytrium sp.* KL2a ; *Thraustochytrium sp.* ONC-T18 ; *Thraustochytrium sp.* PJA10.2 ; *Thraustochytrium sp.* TR1.4 ; *Thraustochytrium sp.* TRR2 ; *Thraustochytrium striatum* ; *Thraustochytrium striatum* ATCC24473 ; *Thraustochytrium striatum* DQ323163 ; *Thraustochytrium striatum* DQ356665 ; *Thraustochytrium visurgense* ; *Ulkenia amoeboidea* SEK 214 ; *Ulkenia profunda* ; *Ulkenia profunda* BUTRBG 111 ; *Ulkenia sp* ; *Ulkenia sp.* ATCC 28207 ; *Ulkenia visurgensis* ; *Ulkenia visurgensis* BURAAA 141 ; *Ulkenia visurgensis* ATCC 28208.

Préférentiellement selon l'invention les Thraustochytrides peuvent être choisis parmi les genres *Aurantiochytrium* et *Schyzochitrium,* préférentiellement parmi les espèces

*Aurantiochytrium mangrovei* CCAP 4062/2 déposée le 20 mai 2014 à la CCAP,
*Aurantiochytrium mangrovei* CCAP 4062/3 déposée le 20 mai 2014 à la CCAP,
*Aurantiochytrium mangrovei* CCAP 4062/4 déposée le 20 mai 2014 à la CCAP,
*Aurantiochytrium mangrovei* CCAP 4062/5 déposée le 20 mai 2014 à la CCAP,
*Aurantiochytrium mangrovei* CCAP 4062/6 déposée le 20 mai 2014 à la CCAP,
*Aurantiochytrium* CCAP 4062/1 déposée le 21 juin 2013 à la CCAP,
*Schizochytrium sp.* CCAP 4087/3 déposée le 20 mai 2014 à la CCAP,
*Schizochytrium sp.* CCAP 4087/1 déposée le 28 février 2012 à la CCAP,
*Schizochytrium sp.* CCAP 4087/4 déposée le 20 mai 2014 à la CCAP et
*Schizochytrium sp.* CCAP 4087/5 déposée le 20 mai 2014 à la CCAP.

Tous les dépôts ont été faits à la CCAP (CULTURE COLLECTION OF ALGAE AND PROTOZOA (CCAP), SAMS Research Services Ltd., Scottish Marine Institute, OBAN, Argyl PA37 1QA United Kingdom) selon les dispositions du traité de Budapest.

Selon une variante préférée de l'invention ladite biomasse peut être une biomasse

- de Thraustochytrides non génétiquement modifiés choisis parmi les genres *Aurantiochytrium* et *Schyzochitrium,* préférentiellement parmi les espèces *Aurantiochytrium mangrovei* CCAP 4062/2 ; *Aurantiochytrium mangrovei* CCAP 4062/3 ; *Aurantiochytrium mangrovei* CCAP 4062/4 ; *Aurantiochytrium mangrovei* CCAP 4062/5 ; *Aurantiochytrium mangrovei* CCAP 4062/6 ; *Aurantiochytrium mangrovei* CCAP 4062/1 ; *Schizochytrium sp.* CCAP 4087/3 ; *Schizochytrium sp.* CCAP 4087/1 ; *Schizochytrium sp.* CCAP 4087/4 ; *Schizochytrium sp.* CCAP 4087/5 ;
- ladite biomasse ayant une quantité substantiellement majoritaire de Thraustochytrides qui n'est pas dégradée, et présentant :
- une teneur en protéines, en poids par rapport au poids de la matière sèche, au moins 35% de protéines, préférentiellement au moins 45 % de protéines, pouvant aller jusqu'à plus de 60 % de protéines, voire plus de 75 % de protéines, en particulier de 45 à 75 % de protéines;
- une teneur en matière grasse, en poids par rapport au poids de la matière sèche, de moins de 20 % préférentiellement de moins de 10 %, très préférentiellement de moins de 7% ; et
- un taux d'humidité avant séchage de 70 à 90 %, préférentiellement de 80% à 85 % ou un taux d'humidité après séchage est de 1% à 10%, préférentiellement de 2% à 7 %.

L'invention a aussi pour objet un procédé de production d'une biomasse telle que décrite précédemment qui comprend :

a) Une première étape de culture des Thraustochytrides dans un milieu de culture approprié et dans des conditions pouvant favoriser la production de protéines à une teneur au moins 35% de protéines, préférentiellement au moins 45 % de protéines, pouvant aller jusqu'à plus de 60 % de protéines, voire plus de 75 % de protéines, en particulier de 45 à 75 % de protéines, et qui, éventuellement, limitent la production de matière grasse à une teneur de moins de 20% de matière grasse en poids par rapport au poids de la matière sèche, préférentiellement moins de 10% de matière grasse, encore plus préférentiellement moins de 7% de matière grasse, jusqu'à l'obtention d'une densité de culture d'au moins 40 g/L en matière sèche, préférentiellement au moins 60 g/L, plus préférentiellement au moins 80 g/L ;
b) une deuxième étape de récupération de la biomasse obtenue à la première étape par séparation de ladite biomasse du milieu de culture ; et, le cas échéant,
c) une troisième étape de séchage de la biomasse récupérée à la deuxième étape.

De manière préférentielle, l'étape a) de culture des Thraustochytrides est mise en oeuvre dans un milieu de culture et dans des conditions appropriées pour favoriser la production de protéines et pour limiter la production en matière grasse.

Selon l'invention, ledit milieu de culture approprié est de préférence un milieu de culture chimiquement défini qui comprend une source de carbone, une source d'azote, une source de phosphore et des sels.

Par "milieu de culture chimiquement défini ", on entend selon l'invention un milieu de culture dans lequel la teneur de chaque élément est connue. Précisément, l'invention vise un milieu pouvant ne pas comporter de matières organiques riches ou complexes. Par matières organiques riches ou complexes on entend des matières organiques non purifiées, se présentant sous la forme de mélanges pour lesquels la composition exacte et les concentrations des divers composants du mélange ne sont pas connues avec exactitude, pas maitrisées, et peuvent présenter une variabilité significative d'un lot à un autre. Comme exemple de matière organique riche ou complexe, on peut citer les extraits de levure ou les peptones qui sont des produits d'une réaction d'hydrolyse de protéines ou encore les matières minérales riches comme par exemple les sels minéraux marins ou autres agents de croissance complexes, n'ayant pas de concentration fixe de chacun de leurs composants.

Selon l'invention ledit milieu défini peut comprendre des sels choisis parmi les sels de calcium, de cobalt, de manganèse, de magnésium, de zinc, de nickel, de cuivre, de potassium, de fer, de sodium, et leurs mélanges.

Avantageusement lesdits sels peuvent être choisis parmi le chlorure de calcium, le chlorure de cobalt, le chlorure de manganèse, le sulfate de magnésium, le sulfate de zinc, le sulfate de nickel, le sulfate de cuivre, le sulfate de potassium, le sulfate ferreux, le sulfate de potassium, le molybdate de sodium, le sélénite de sodium, le chlorure de sodium et leurs mélanges.

Selon une variante de l'invention et selon les souches employées, le milieu peut également comprendre du chlorure de sodium (NaCl), notamment pour certaines souches d'origine marine.

Selon cette variante, on peut citer à titre d'exemple de souches marines pouvant admettre un milieu de culture pouvant comprendre du chlorure de sodium, les souches de *Schizochytrium sp.,* en particulier *Schizochytrium sp.* CCAP 4062/3.

Selon une autre variante de l'invention et selon les souches employées, le milieu peut ne pas comprendre du chlorure de sodium (NaCl), à tout le moins comprendre une quantité de chlorure de sodium très faible, ayant moins de 3,5 g/L, de préférence moins de 1 g/L, plus préférentiellement moins de 10 mg/L d'ions de sodium et moins de 1 g/L, de préférence moins de 500 mg/L, plus préférentiellement 200 mg/L d'ions de chlorure.

Selon cette variante, on peut citer à titre d'exemple de souches pouvant admettre un milieu de culture pouvant ne pas comprendre du chlorure de sodium (NaCl), à tout le moins comprendre une quantité de chlorure de sodium très faible, les souches *d'Aurantiochytrium mangrovei,* en particulier la souche *Aurantiochytrium mangrovei* CCAP 4062/5.

Selon l'invention, la source de carbone dudit milieu défini peut-être un (ou des) glucide(s), un (ou des) acétate(s), un ou des alcools, une ou des molécules complexes, ou tout mélange, en toute proportion d'au moins 2 de ces sources.

Selon l'invention, ladite source d'azote dudit milieu défini peut-être choisie parmi un (ou des) sel(s) de nitrate, un (ou des) sel(s) de glutamate, un (ou des) sel(s) d'ammonium, l'urée, l'ammoniaque ou tout mélange, en toute proportion d'au moins 2 de ces sources.

Selon l'invention, la source de phosphore dudit milieu défini peut-être choisie parmi l'acide phosphorique, les sels de phosphate, avantageusement de l'hydrogénophosphate de sodium ($Na_2HPO_4$), ou du dihydrogénophosphate de sodium ($NaH_2PO_4$), ou du dihydrogénophosphate de potassium ($KH_2PO_4$), ou de l'hydrogénophosphate de potassium ($K_2HPO_4$), ou tout mélange, en toute proportion d'au moins 2 de ces sources.

Selon une variante de l'invention ledit milieu de culture pourra comprendre du chlorure de magnésium, avantageusement sous forme de tetrahydrate ($MgCl_2\,4H_2O$) ; du chlorure de calcium, avantageusement sous forme dihydrate ($CaCl_2\,2H_2O$) ; du chlorure de cobalt hexahydrate ($CoCl_2\,6H_2O$) ; du chlorure de manganèse (II) tetrahydrate ($MnCl_2\,4H_2O$) ; du sulfate de magnésium heptahydrate (MgSO4, 7H2O) ; du sulfate de zinc heptahydrate (ZnSO4, 7H2O) ; du sulfate de nickel hexahydrate ($NiSO_4\,6H_2O$) ; du sulfate de cuivre pentahydrate ($CuSO_4\,5H_2O$) ; du sulfate de potassium ($K_2SO_4$) ; du sulfate ferreux heptahydrate ($FeSO_4,\,7H_2O$) ; de l'acide borique ($H_3BO_3$) ; de l'acide éthylène diamine tétraacétique sous forme disodique dihydrate ($Na_2EDTA,\,2H_2O$) ; du molybdate de sodium dihydrate, ($Na_2MoO_4,\,2H_2O$) ; du sélénite de sodium, ($Na_2SeO_3$) ; à titre de vitamine de la thiamine, de la cobalamine ou vitamine B12, du panthoténate ou vitamine B5 ; une source de carbone ; une source d'azote ; une source de phosphore.

Selon une forme préférée de l'invention, dans ledit milieu de culture le chlorure de magnésium peut être à une concentration comprise entre 0,008 et 0,012 g/L, avantageusement entre 0,009 et 0,011 g/L ; le chlorure de calcium peut être à une concentration comprise entre 0,40 et 0,70 g/L, avantageusement entre 0,50 et 0,60 g/L ; le chlorure de cobalt hexahydrate peut être à une concentration comprise entre 0,00008 et 0,00013 g/L, avantageusement entre 0,00009 et 0,00012 g/L ; le chlorure de manganèse (II) tetrahydrate peut être à une concentration comprise entre 0,008 et 0,013, avantageusement entre 0,009 et 0,012 g/L ; le sulfate de magnésium heptahydrate peut être à une concentration comprise entre 6 et 10 g/L, avantageusement entre 7 et 9 g/L ; le sulfate de zinc heptahydrate peut être à une concentration comprise entre 0,008 et 0,013, avantageusement 0,009 et 0,012 g/L ; le sulfate de nickel hexahydrate peut être à une concentration comprise entre 0,004 et 0,007 g/L, avantageusement 0,005 et 0,006 g/L ; le sulfate de cuivre pentahydrate peut être à une concentration comprise entre 0,005 et 0,009 g/L, avantageusement entre 0,006 et 0,008 g/L ; le sulfate de potassium peut être à une concentration comprise entre 0,5 et 3,5 g/L, avantageusement entre 1 et 3 g/L ; le sulfate ferreux heptahydrate peut être à une concentration comprise entre 0,03 et 0,05 g/L, avantageusement entre 0,035 et 0,045 g/L ; l'acide borique peut être à une concentration comprise entre 0,0155 et 0,0195 g/L, avantageusement entre 0,0165 et 0,0185 g/L ; l'acide éthylène diamine tétraacétique sous forme disodique dihydrate peut être à une concentration comprise entre 0,10 et 0,14 g/L, avantageusement entre 0,11 et 0,13 g/L ; le molybdate de sodium dihydrate peut être à une concentration comprise entre 0,00001 et 0,0003 g/L, avantageusement entre 0,00005 et 0,0002 g/L ; le sélénite de sodium peut être à une concentration comprise entre 0,00000015 et 0,000019 g/L, avantageusement entre 0,00000016 et 0,00000018 g/L ; la thiamine peut être à une concentration comprise entre 0,015 et 0,05 g/L, avantageusement entre 0,025 et 0,04 g/L ; la cobalamine ou vitamine B12 peut être à une concentration comprise entre 0,0004 et 0,00065 g/L, avantageusement entre 0,00045 et 0,00060 g/L ; le panthoténate ou vitamine B5 peut être à une concentration comprise entre 0,008 et 0,013, avantageusement entre 0,009 et 0,012 g/L ; la source de carbone peut être à une concentration comprise entre 45 et 65 g/L, avantageusement entre 50 et 60 g/L ; la source d'azote peut être à une concentration comprise entre 7 et 11 g/L, avantageusement entre 8 et 10 g/L ; la source de phosphore peut être à une concentration comprise entre 2 et 6 g/L, avantageusement entre 3 et 5 g/L.

Très préférentiellement selon l'invention, dans ledit milieu de culture le chlorure de magnésium est à une concentration de 0,0108 g/L, le chlorure de calcium est à une concentration de 0,55 g/L ; le chlorure de cobalt hexahydrate ($CoCl_2$ 6$H_2$O) est à une concentration de 0,000108 g/L ; le chlorure de manganèse (II) tetrahydrate est à une concentration de 0,0108 g/L ; du sulfate de magnésium heptahydrate est à une concentration de 8,01 g/L ; le sulfate de zinc heptahydrate est à une concentration de 0,0108 g/L ; du sulfate de nickel hexahydrate est à une concentration de 0,0056 g/L ; le sulfate de cuivre pentahydrate est à une concentration de 0,0072 g/L ; le sulfate de potassium est à une concentration de 2,09 g/L ; le sulfate ferreux heptahydrate est à une concentration de 0,04 g/L ; l'acide borique est à une concentration comprise entre 0,0155 et 0,0195 g/L de 0,0175g/L ; l'acide éthylène diamine tétraacétique sous forme disodique dihydrate est à une concentration de 0,12 g/L ; le molybdate de sodium dihydrate, est à une concentration de 0,000108 g/L ; le sélénite de sodium, est à une concentration de 0,000000173 g/L ; la thiamine est à une concentration de 0,032 g/L ; la cobalamine ou vitamine B12 est à une concentration de 0,00052 g/L ; le panthoténate ou vitamine B5 est à une concentration de 0,0108 g/L ; la source de carbone est à une concentration de 55 g/L ; la source d'azote est à une concentration de 9 g/L ; la source de phosphore est à une concentration de 4 g/L.

Selon l'invention, la première étape a) de culture du procédé peut être réalisée en mode discontinu dit "batch", en mode semi-continu dit "fed batch" ou en mode continu.

Selon un mode particulier de réalisation de l'invention, la première étape est divisée en 2 sous-étapes, une première sous-étape a1) de croissance dans le milieu de culture approprié suivie d'une deuxième sous-étape a2) de production dans laquelle on peut ajouter au milieu de culture, simultanément ou successivement, une ou plusieurs solution(s) d'enrichissement en source de carbone, en source d'azote et/ou en source de phosphore de manière à maintenir dans le milieu de culture des teneurs en azote et en phosphore non limitantes pour la croissance.

Selon un mode préféré de réalisation, la sous-étape a1) de croissance est mise en oeuvre jusqu'd'une concentration en source de carbone, plus particulièrement en glucose inférieure à 20 g/L.

Selon un autre mode de réalisation, la sous-étape a1) de croissance est mise en oeuvre jusqu'à l'obtention d'une densité de culture d'au moins 20 g/L, préférentiellement d'au moins 40 g/L, plus préférentiellement au moins 60 g/L, encore plus préférentiellement au moins 80 g/L

La teneur en source d'azote non limitante dans l'étape a2) est avantageusement comprise entre 0,5 et 5 g/L, préférentiellement entre 0,5 et 2 g/L et la teneur en source de phosphore non limitante est avantageusement comprise entre 0,5 et 5 g/L, préférentiellement 0,5 et 2 g/L. La teneur en source de carbone recherchée pour cette étape a2) pourra être comprise entre 0 et 200 g/L, notamment entre 5 voire 10 et 50 g/L. De manière préférentielle la teneur en source de carbone dans la sous-étape a2) est comprise entre 0 et 50 g/L, plus préférentiellement entre 0 et 10 g/L.

De manière préférentielle, la culture dont la première étape a) est divisée en deux sous-étapes a1) et a2) telles que définies ci-dessus est réalisée en mode semi-continu dit "fed batch".

Les méthodes et milieux de culture appropriés pour permettre la croissance de Thraustochytrides à des densités de plus de 20 g/L, et en particulier de plus de 80 g/L sont bien connues de l'homme du métier.

Selon l'invention, la culture peut être réalisée par toute technique de culture connue, par exemple en fioles ou en réacteur, mais aussi en fermenteurs ou encore dans tout contenant apte à la croissance de protistes, particulièrement de Thraustochytrides, comme par exemple des bassins de type "raceway", à condition que ladite technique permette de mettre en oeuvre les conditions de culture requises.

De manière préférentielle, la culture est réalisée en fermenteurs selon les méthodes connues de culture des protistes en fermenteurs.

Selon l'invention la deuxième étape b) du procédé permettant la récupération de ladite biomasse peut être réalisée dans des conditions appropriées pour obtenir une biomasse pouvant présenter le taux d'humidité recherché.

Ladite récupération des protistes, peut être réalisée par toute technique permettant la récupération de la biomasse, notamment les méthodes de filtration, gravimétrique ou sous pression réduite, de centrifugation, de décantation, ou bien encore des méthodes de précipitation suivie d'une filtration gravimétrique.

L'invention a aussi pour objet une biomasse susceptible d'être obtenue par le procédé selon l'invention tel que décrit précédemment dans toutes ses variantes.

L'invention a encore pour objet l'utilisation d'une biomasse telle que décrite précédemment dans les domaines cosmétiques, pharmaceutiques, alimentaires humain ou animal.

Elle concerne en particulier l'utilisation d'une biomasse de Thraustochytrides selon l'invention, telle que décrite ci-dessus et ci-après, pour l'amélioration des performances des animaux. Cette amélioration de la performance peut être évaluée, en particulier, par la mesure de la consmooationde consommation, du gain de poids ou encore du « Feed Conversion Ratio » (rapport de conversion de l'aliment).

On pourra distinguer dans l'alimentation animale, l'alimentation des animaux d'élevage, en particulier en élevage industriel et celle des animaux domestiques ou encore des animaux de compagnie ou des animaux dits de « loisir » , comme les poissons d'aquarium ou les oiseaux de volière ou en cage.

Par « animal d'élevage » on entend notamment les animaux de pacage (notamment les bovins élevés pour la viande, le lait, le fromage et le cuir ; les ovins élevés pour la viande, la laine et le fromage ; les caprins), les porcins, les lapins, les volailles (les poulets, les poules, les dindes, les canards, les oies et autres), les équidés (poneys, chevaux, poulains), destinés à supporter des activités humaines (transport, loisirs) ou leur alimentation, les animaux aquatiques (par exemple les poissons, les crevettes, les huîtres et les moules). On pourra toutefois distinguer l'alimentation des poissons jusqu'au stade d'alevins, et celles des poissons élevés, y compris les aliments et compositions alimentaires qui leur sont destinées.

On les distinguera des animaux domestiques, animaux de compagnie ou animaux de loisir qui comprennent également des mammifères, ruminants ou non, des oiseaux ou des poissons. Ils comprennent en particulier les chiens et les chats.

L'invention a aussi pour objet un aliment, ou composition alimentaire, pour l'homme ou les animaux, pouvant comprendre une biomasse selon l'invention telle que décrite précédemment. On entend par « aliment » toute composition qui peut servir à la nourriture de l'homme ou des animaux, en particulier les animaux d'élevage.

Selon l'invention l'aliment peut ne comprendre que de la biomasse, séchée ou non, transformée ou non, ou de la biomasse, séchée ou non, transformée ou non, mélangée à tout autre additif, véhicule ou support, utilisé dans le domaine de l'alimentation humaine ou animale, comme par exemple les conservateurs alimentaires, les colorants, les exhausteurs de goût, les régulateurs du pH, ou encore des additifs pharmaceutiques comme par exemple des hormones de croissance, des antibiotiques.

La présente invention concerne en particulier des aliments pour animaux et plus particulièrement pour les animaux d'élevage. Ces aliments se présentent habituellement sous la forme de farines, de granulés ou de soupe dans lesquels est incorporée la biomasse selon l'invention. On entend par "aliment" tout ce qui peut servir à la nourriture des animaux.

Pour l'élevage intensif des animaux, les aliments peuvent comprendre, en plus de la biomasse algale, une base nutritionnelle et des additifs nutritionnels. L'essentiel de la ration alimentaire de l'animal est alors constituée par la "base nutritionnelle" et la biomasse algale. Cette base est constituée à titre d'exemple par un mélange de céréales, de protéines et de matières grasses d'origine animale et/ou végétale.

Les bases nutritionnelles pour animaux sont adaptées à l'alimentation de ces animaux et sont bien connues de l'homme du métier. Dans le cadre de la présente invention, ces bases nutritionnelles comprennent par exemple du maïs, du blé, du pois et du soja. Ces bases nutritionnelles sont adaptées aux besoins des différentes espèces animales auxquelles elles sont destinées. Ces bases nutritionnelles peuvent déjà contenir des additifs nutritionnels comme des vitamines, des sels minéraux et des acides aminés.

Les additifs utilisés en alimentation animale peuvent être ajoutés pour améliorer certaines caractéristiques des aliments, par exemple pour en relever le goût, pour rendre plus digestes les matières premières des aliments pour animaux ou protéger les animaux. Ils sont fréquemment utilisés dans les élevages intensifs de grande envergure.

Les additifs utilisés en alimentation animale se déclinent, notamment, dans les souscatégories suivantes (source EFSA):

- additifs technologiques: par exemple, conservateurs, antioxydants, émulsifiants, stabilisateurs, régulateurs d'acidité et additifs pour l'ensilage;
- additifs sensoriels: par exemple, arômes, colorants,
- additifs nutritionnels: par exemple, vitamines, aminoacides et oligo-éléments;
- additifs zootechniques: par exemple, améliorateurs de digestibilité, stabilisateurs de flore intestinale;
- coccidiostatiques et histomonostatiques (antiparasitaires).

Dans un mode de réalisation, l'invention concerne des aliments pour animaux d'élevage comprenant entre 1 et 60 %, de préférence entre 1 et 20%, de façon tout à fait préférentielle entre 3 et 8 % d'une biomasse séchée ou obtenue par le procédé selon la l'invention.

Dans un autre mode de réalisation, l'invention concerne des aliments pour animaux d'élevage comprenant entre 1 et 40 %, de préférence entre 5 et 10% d'une biomasse non séchée obtenue par le procédé de l'invention.

Selon un mode particulier de réalisation de l'invention, l'aliment est destiné aux animaux d'élevage, en particulier les bovins, les ovins, les porcins, les lapins, les volailles et les équidés.

Selon un autre mode particulier de l'invention, l'aliment est destiné aux animaux aquatiques, en particulier aux poissons, au moins jusqu'au stade alevins, voire y compris les poissons d'élevage.

Selon un autre mode particulier de réalisation de l'invention, l'aliment est destiné aux animaux domestiques, animaux de compagnie et/ou animaux de loisirs.

Enfin, selon un autre mode de réalisation de l'invention, la composition alimentaire est destinée à l'homme.

L'invention à également pour objet une composition cosmétique ou pharmaceutique pour l'homme ou les animaux comprenant une biomasse selon l'invention telle que décrite précédemment.

Selon l'invention la composition cosmétique ou pharmaceutique peut ne comprendre que de la biomasse, séchée ou non, transformée ou non, ou de la biomasse, séchée ou non, transformée ou non, mélangée à tout autre additif, véhicule ou support, utilisé dans le domaine de la cosmétique ou de la pharmacie comme par exemple les conservateurs, les colorants, les régulateurs du pH.

L'invention a aussi pour objet l'utilisation de la biomasse telle que décrite précédemment en thérapie, ainsi que dans la prévention et le traitement de la malnutrition.

Figure 1 : Schéma expérimental de la mesure de l'Energie Métabolisable Apparente (AME) chez le poulet 23 jours ; (essai 14ALG069)

Figure 2 : Schéma expérimental du test de choix et de consommation chez le poussin (7 et 9 jours) ; (essai 14ALG081)

Figure 3 : Mesure de la consommation d'aliments à 7 jours d'âge dans des conditions de choix (aliment maïs-soja vs aliments substitués à 5, 10 et 15% de la microalgue testée) (essai 14ALG081)

Figure 4 : Mesure de la consommation d'aliments à 9 jours d'âge dans des conditions de choix (aliment maïs-soja vs aliments substitués à 5, 10 et 15% de la microalgue testée) (essai 14ALG081)

Figure 5 : Mesure de la consommation relative moyenne d'aliments sur les 2 tests (à 7j et 9j) dans des conditions de choix (aliment maïs-soja vs aliments substitués à 5, 10 et 15% de la microalgue testée) (essai 14ALG081)

Figure 6 : Mesure de la performance de croissance (consommation, gain de poids, indice de consommation) des poussins de 0 à 7 jours alimentés d'un régime contrôle maïs-soja vs des aliments substitués à 5, 10 et 15% de la microalgue testée (non en conditions de choix) (essai 14ALG081)

Figure 7 : Mesure de la performance de croissance (consommation, gain de poids, indice de consommation) des poussins de 0 à 9 jours alimentés d'un régime contrôle maïs-soja vs des aliments substitués à 5, 10 et 15% de la microalgue testée (non en conditions de choix) (essai 14ALG081)

## EXEMPLES

D'autres aspects et caractéristiques de l'invention pourront apparaitre à la lecture des exemples qui suivent ainsi que dans les figures annexées lesquelles décrivent

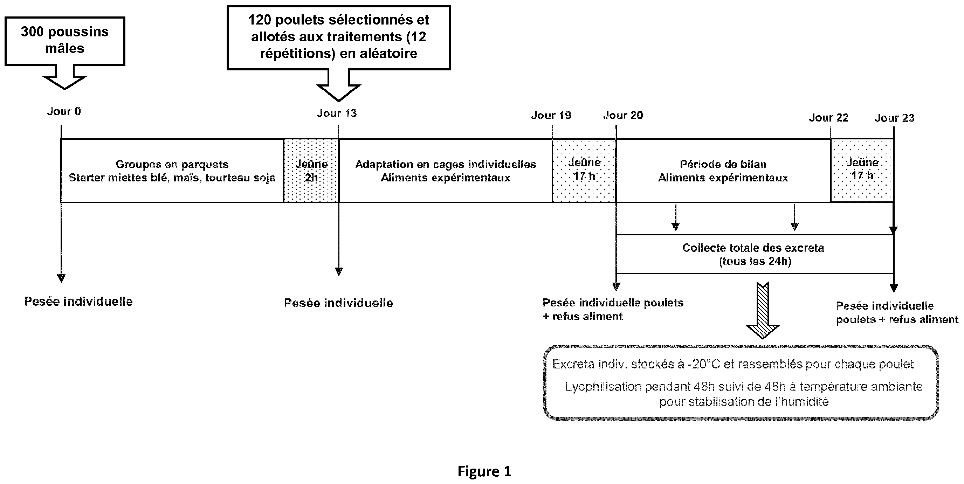

- pour la figure 1, le schéma expérimental de la mesure de l'Energie Métabolisable Apparente (AME) chez le poulet 23 jours ; (essai 14ALG069). Environ 300 poussins males (Ross PM3) de 1 jour ont été installés dans un même parquet et nourris avec un aliment démarrage standard à base de blé, de maïs et de tourteau de soja. A 13 jours d'âge les poulets sont mis à jeun 2h avant d'être pesés et répartis par classe de poids. 120 poulets sont ainsi sélectionnés, installés dans des cages métaboliques individuelles et assignés à l'un des traitements expérimentaux selon leur poids (20 répétitions par régime).

Les aliments et l'eau sont distribués *ad libitum* tout au long de l'essai. Les aliments expérimentaux sont présentés en granulés de diamètre 3,2 mm. Après une période d'adaptation de 7 jours aux régimes et aux cages métaboliques, une période de collecte totale des excrétas, encadrée par des jeûnes de 17h, a été menée pendant trois jours : de j20 à j23. Les excrétas sont collectés individuellement chaque jour, cumulés et stockés à - 20°C. A la fin de l'essai les excrétas collectés sont lyophilisés puis laissés à température ambiante pour une phase de reprise en eau (48h) afin de stabiliser le contenu en humidité avant pesée, broyage (0,5 mm) et analyses.

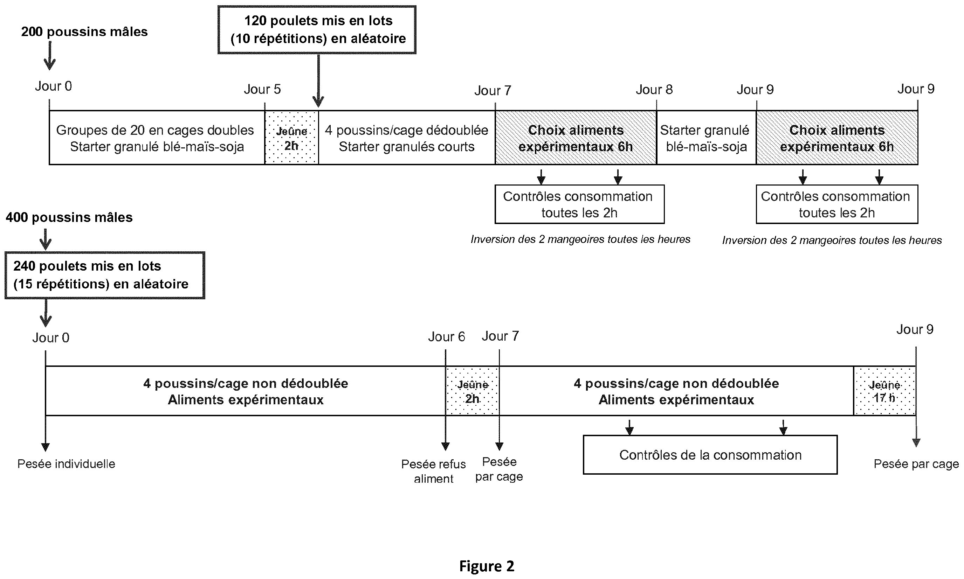

- Pour la figure 2, le schéma expérimental du test de choix et du test de consommation chez le poussin (7 et 9 jours) ; (essai 14ALG081). Deux essais expérimentaux ont été conduits en parallèle.

1) Essai 1 - test de choix : Pour l'essai avec choix de l'aliment, environ 200 poussins mâles (Ross PM3) de 1 jour ont été installés par groupe de 20 environ dans des cages métaboliques dédoublées et nourris avec un aliment de démarrage standard (starter) à base de blé, de maïs et de tourteau de soja. A 6 jours d'âge les poulets sont mis à jeun 2h avant d'être pesés et répartis par classes de poids. Cent-vingt poulets sont ainsi sélectionnés, installés par groupes de 4 dans 30 cages métaboliques dédoublées et assignés au jour 7 à l'un des traitements expérimentaux selon leur poids (10 répétitions par traitement). Chaque cage contient 2 mangeoires contenant des aliments différents, correspondant aux 3 traitements expérimentaux suivants :

   o Traitement 1 : starter maïs-soja (mangeoire 1) et microalgue 5% (mangeoire 2)
   o Traitement 2 : starter maïs-soja (mangeoire 1) et microalgue 10% (mangeoire 2)
   o Traitement 3 : starter maïs-soja (mangeoire 1) et microalgue 15% (mangeoire 2)

Les aliments et l'eau sont distribués *ad libitum* tout au long de l'essai. Les aliments expérimentaux sont présentés en granulés de diamètre 3,2 mm. Une mesure des consommations est réalisée à T0 + 1h, T0 + 2h, T0 + 3 h, T0 + 4h, et T0 + 6h au 7ième et 9ième jour d'âge, avec inversion des mangeoires toutes les heures dans les cages. Entre les deux mesures de consommation (jour 8), les animaux reçoivent l'aliment démarrage blé-maïs-soja.

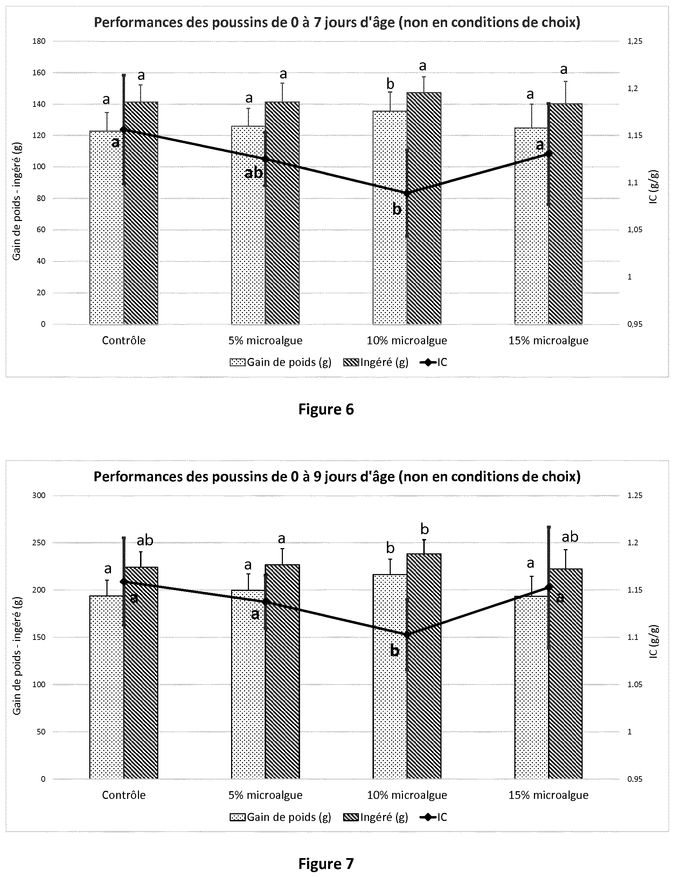

2) Essai 2- mesure de consommation : dans un deuxième test les poussins n'ont accès qu'à un seul type d'aliment, supplémenté ou non avec la microalgue (figure 6). Environ 400 poussins males (Ross PM3) de 1 jour ont été pesés et répartis par classe de poids. Deux-cent-quarante animaux sélectionnés sont placés par 4 au jour 1 dans 60 cages non dédoublées par bloc de poids homogène (15 blocs de 4 cages) avec une mangeoire par cage, chacune avec un aliment expérimental (15 répétitions par aliment). Dans chaque cage, les 4 poussins sont identifiés individuellement. Les aliments et l'eau sont distribués *ad libitum* tout au long de l'essai. Les aliments expérimentaux sont l'aliment de démarrage (starter) maïs-soja supplémenté avec 0, 5, 10 ou 15% de microalgue, également utilisés dans l'essai 1 avec choix de l'aliment. Les poids vifs individuels sont mesurés à 1, 7 et 9 jours d'âge. Les refus sont pesés et les consommations par cage mesurées à 7 et 9 jours d'âge. De 0 à 7 jours d'âge, les animaux nourris avec l'aliment contenant 10 % de microalgue ont un gain de poids significativement amélioré par rapport au contrôle et aux deux autres régimes supplémentés.

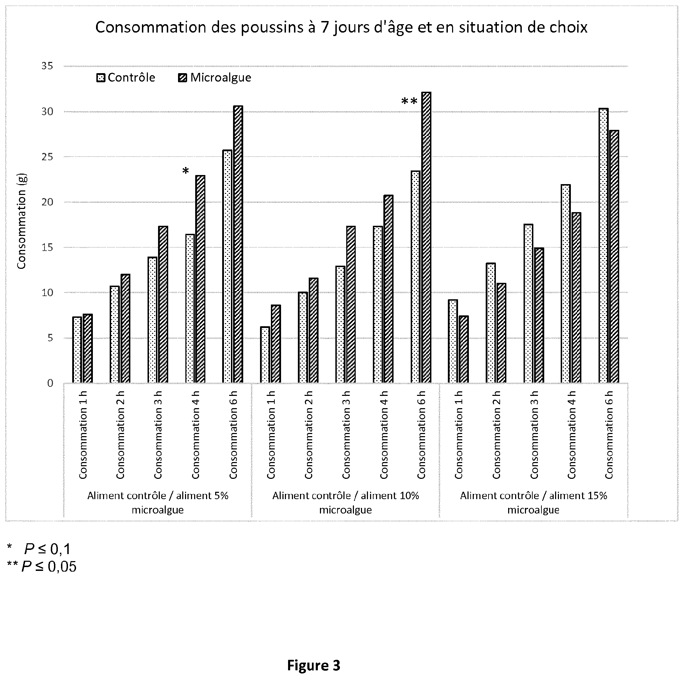

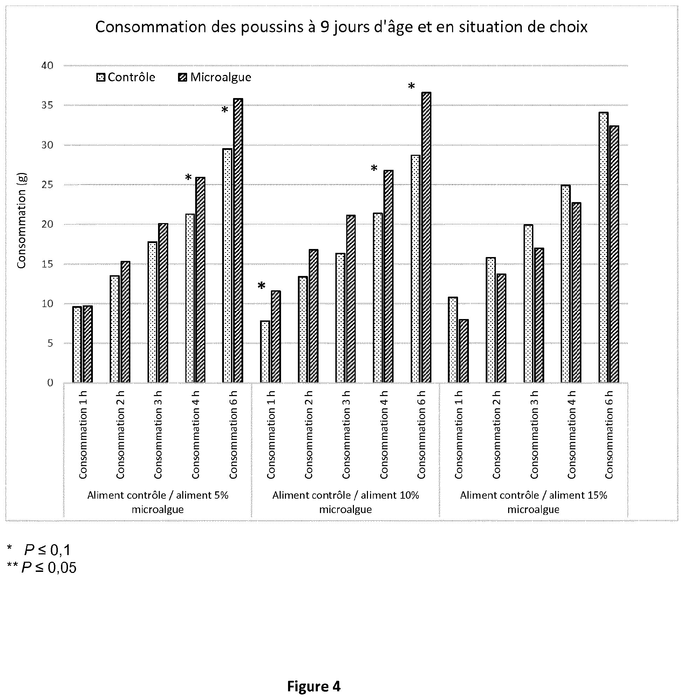

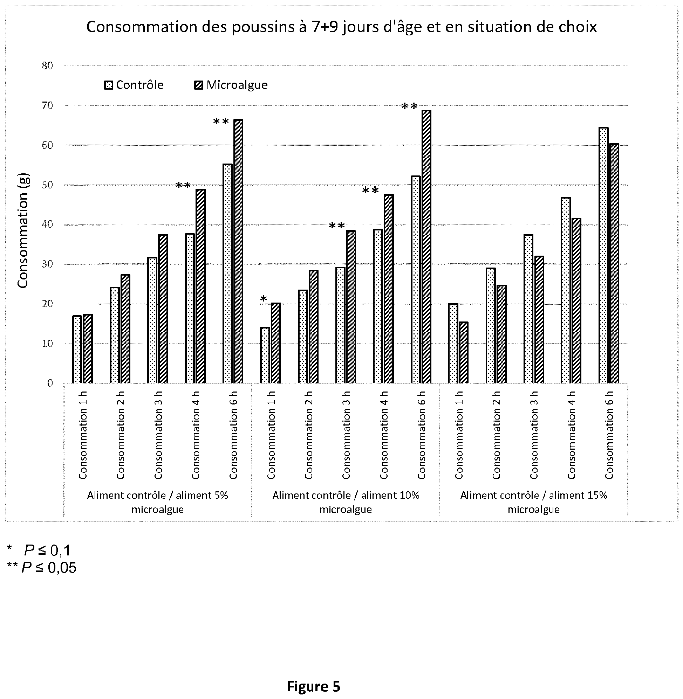

- Pour la figure 3, la mesure de la consommation d'aliments à 7 jours d'âge dans des conditions de choix (aliment maïs-soja vs aliments substitués à 5, 10 et 15% de la microalgue testée) (essai 14ALG081) ;
- Pour la figure 4, la mesure de la consommation d'aliments à 9 jours d'âge dans des conditions de choix (aliment maïs-soja vs aliments substitués à 5, 10 et 15% de la microalgue testée) (essai 14ALG081) ;
- Pour la figure 5, la mesure de la consommation relative moyenne d'aliments sur les 2 tests (à 7j et 9j) dans des conditions de choix (aliment maïs-soja vs aliments substitués à 5, 10 et 15% de la microalgue testée) (essai 14ALG081) ;
- Pour la figure 6, la mesure de la performance de croissance (consommation, gain de poids, indice de consommation) des poussins de 0 à 7 jours alimentés d'un régime contrôle maïs-soja vs des aliments substitués à 5, 10 et 15% de la microalgue testée (non en conditions de choix) (essai 14ALG081) ;
- Pour la figure 7, la mesure de la performance de croissance (consommation, gain de poids, indice de consommation) des poussins de 0 à 9 jours alimentés d'un régime contrôle maïs-soja vs des aliments substitués à 5, 10 et 15% de la microalgue testée (non en conditions de choix) (essai 14ALG081).

**EXEMPLE 1 - Culture des souches**

**Précultures des souches** :

Une préculture *d'Aurantiochytrium mangrovei* est réalisée sur table d'agitation (140 t/min) en enceinte thermostatée (26 °C), en milieu de préculture, contenant 4 g d'extrait de levure comme source d'azote, et de 30 g de glucose comme source de carbone. Après 48 heures d'incubation les cellules sont centrifugées 5 minutes à 3000 g et le culot de cellules rincé avec du milieu de préculture ne contenant ni extrait de levure ni aucune autre source d'azote minérale ou organique. Cette opération a pour but d'éviter tout apport de Na⁺ dans la culture principale via l'ajout d'extrait de levure. La préculture correspond à 1/100 (v/v) du volume de culture de la solution principale. Dans le cas de la souche de *Schizochytrium* sp. CCAP 4062/3, 27 g/l de NaCl sont ajoutés à ce milieu.

**Suivi des cultures** :

La concentration en biomasse totale est suivie par mesure de la masse sèche (filtration sur filtre GF/F, Whatman, puis séchage en étuve, à 105 °C, pendant 24 h minimum avant pesée).

Les analyses en contenus lipidiques totaux et des FAMEs sont réalisées selon les méthodes classiquement décrites dans la littérature (Folch et col., A simple method for the isolation and purification of total lipids from animal tissues. Folch J, et col., J Biol Chem. 1957 May;226(1):497-509. ; Yokoyama et col., Taxonomic rearrangement of the genus Schizochytrium sensu lato based on morphology, chemotaxonomic characteristics, and 18S rRNA gene phylogeny (Thraustochytriaceae, Labyrinthulomycetes): emendation for Schizochytrium and érection of Aurantiochytrium and Oblongichytrium gen. nov.; Mycoscience, 2007 Vol. 48, pp. 199-211).

### Exemple 1.1 (comparatif)

Des cultures *d'Aurantiochytrium mangrovei* CCAP 4062/5 sont réalisées dans des fermenteurs (bioréacteurs) de 1 à 2 L utiles avec automates dédiés et supervision par station informatique.

La composition du milieu de culture ainsi que celles correspondant aux solutions d'ajout sont les suivantes :

| Solution principale | Concentration (g/L) |
|---|---|
| KCl | 0,36 |
| $H_3BO_3$ | 0,175 |
| $MgSO_4, 7H_2O$ | 6,750 |
| $CaCl_2, 2H_2O$ | 0,55 |
| $KNO_3$ | 0,04667 |
| $KH_2PO_4, 7H_2O$ | 0,30940 |
| $Na_2EDTA, 2H_2O$ | $3,094.10^{-3}$ |
| $ZnSO_4.7H_2O$ | $7,3.10^{-5}$ |
| $CoCl_2.6H_2O$ | $1,6.10^{-5}$ |
| $MnCl_2.4H_2O$ | $5,4.10^{-4}$ |
| $Na_2MoO_4, 2H_2O$ | $1,48.10^{-6}$ |
| $Na_2SeO_3$ | $1,73.10^{-6}$ |
| $NiSO_4.6H_2O$ | $2,98.10^{-6}$ |
| $CuSO_4.5H_2O$ | $9,8.10^{-6}$ |
| EDTA-Fe | 0,03 |
| **Carbone** | **g/L** |
| Glucose | 55 |
| **Azote** | **g/L** |
| $(NH4)_2SO_4$ | 7 |
| **Ajout après autoclave** | |
| **Vitamines** | **g/L** |
| Thiamine | $8,0.10^{-3}$ |
| Vitamine B12 | $1,3.10^{-4}$ |
| Panthothénate | $2,7.10^{-3}$ |

| Solution d'ajout 1 | Concentration (g/L) |
|---|---|
| $K_2SO_4$ | 31,9 |
| $MgSO_4, 7H_2O$ | 25,8 |
| $KH_2PO_4, 7H_2O$ | 61,38 |

(suite)

| Solution d'ajout 1 | Concentration (g/L) |
|---|---|
| $FeSO_4, 7H_2O$ | 0,61 |
| $(NH4)_2SO_4$ * | 138,24 |
| $MnCl_2\ 4H_2O$ | 0,165 |
| $ZnSO_4.7H_2O$ | 0,165 |
| $CoCl_2.6H_2O$ | $1,6.10^{-3}$ |
| $Na_2MoO_4, 2H_2O$ | $1,6.10^{-3}$ |
| $CuSO_4, 5H_2O$ | 0,11 |
| $NiSO_4, 6H_2O$ | $8,6.10^{-2}$ |
| $Na_2EDTA, 2H_2O$ | 1,81 |
| Thiamine | 0,49 |
| Vitamine B12 | $8,0.10^{-3}$ |
| Panthothénate | 0,1656 |
| **Solution d'ajout 2** | **Concentration g/L** |
| Glucose | 750 |
| $KH_2PO_4$ | 6,4 |
| $(NH_4)2SO_4$* | 34 |

Le système est régulé à pH 6 via l'ajout de base (NaOH, ou KOH) et/ou d'acide (solution d'acide sulfurique). La température de culture a été fixée à 26°C. La pression d'oxygène dissous a été régulée dans le milieu tout au long de la culture, par la vitesse d'agitation (250 - 1200 t/min), le débit d'air (0,25 - 1 vvm), voire le débit d'oxygène (0,1 - 0,5 vvm). Les paramètres de régulation, intégrés dans l'automate de supervision, ont permis de maintenir une $pO_2$ constante comprise entre 5% et 30%. Le temps de culture a été compris entre 20 et 100 heures, de préférence entre 25 et 96 heures, par exemple 50 heures.

En cours de culture, 3 ajouts de solution d'ajout 1 ont été effectuées, ainsi que des ajouts de la solution 2 afin de maintenir des concentrations en glucose comprises entre 200 mM et 500 mM.

### Exemple 1.2 (invention)

Des cultures *d'Aurantiochytrium mangrovei* CCAP 4062/5 sont réalisées dans des fermenteurs comme essentiellement décrit dans l'Exemple1.1.

La modification de la procédure se fait sur le mode de régulation pH par ajout d'ammoniaque ($NH_4OH$) pour éviter l'apport important de $Na^+$ ou de $K^+$ liées à la régulation pH par la soude ou la potasse, et qui pourrait être gênant pour la valorisation des produits pour l'alimentation animale. Une partie de l'azote nécessaire à la culture des cellules étant apporté via la régulation du pH par l'ammoniaque ($NH_4OH$).

Le milieu utilisé pour cet exemple est décrit dans le tableau 1 ci-dessous. Contrairement au milieu décrit l'exemple 1.1, ce milieu chimiquement défini permet de soutenir la croissance sur l'ensemble de la culture sans limitation nutritionnelles.

Tableau 1

| | Concentration (g/L) |
|---|---|
| chlorure de magnésium | $1,08.10^{-2}$ |
| chlorure de calcium | 0,55 |
| chlorure de cobalt hexahydrate ($CoCl_2\ 6H_2O$) | $1,08.10^{-4}$ |
| chlorure de manganèse (II) tetrahydrate | $1,08.10^{-2}$ |
| sulfate de magnésium heptahydrate | 8,01 |

(suite)

| | Concentration (g/L) |
|---|---|
| sulfate de zinc heptahydrate | $1,08.10^{-2}$ |
| sulfate de nickel hexahydrate | $5,60.10^{-3}$ |
| sulfate de cuivre pentahydrate | $7,20.10^{-3}$ |
| sulfate de potassium | 2,09 |
| sulfate ferreux heptahydrate | $4,00.10^{-2}$ |
| acide borique | $1,75.10^{-2}$ |
| acide éthylène diamine tétraacétique disodique dihydrate | 0,12 |
| molybdate de sodium dihydrate | $1,08.10^{-4}$ |
| sélénite de sodium | $1,73.10^{-7}$ |
| thiamine | $3,20.10^{-2}$ |
| cobalamine ou vitamine B12 | $5,20.10^{-4}$ |
| Panthoténate ou vitamine B5 | $1,08.10^{-2}$ |
| Glucose (source de carbone) | 55,00 |
| Sulfate d'ammonium (source d'azote) | 9,00 |
| dihydrogénophosphate de potassium (source de phosphore) | 4,00 |
| Antimousse | 0,40 |

Les paramètres physicochimiques sont contrôlés durant la culture grâce à des régulateurs intégrés, avec un maintien du pH autour d'une consigne de 5, une régulation de la température à 30°C, et un maintien de la $pO_2$ autour d'une consigne de 30% jusqu'à atteinte des valeurs maximales d'agitation et de débit d'air.

Pendant la culture des ajouts du milieu d'ajout tel que décrit au tableau 2 ci-dessous, sont réalisés de sorte à maintenir la concentration en glucose dans le milieu de culture comprise entre 20g/L et 50g/L.

Tableau 2

| Glucose (source de carbone) | 688 g/L |
|---|---|
| Sulfate d'ammonium (source d'azote) | 76 g/L |
| dihydrogénophosphate de potassium (source de phosphore) | 39 g/L |

**Exemple 1.3 (invention)**

Des cultures d'*Aurantiochytrium mangrovei* CCAP 4062/5 sont réalisées dans des fermenteurs dans des conditions de culture comme essentiellement décrit dans l'Exemple 1.1.

La modification de la procédure porte sur les milieux et sur leur mode d'ajout.

Les cultures sont démarrées en mode batch sur le milieu présenté dans le tableau 3 ci-dessous.

Tableau 3

| | Composé | C° milieu initial |
|---|---|---|
| Glucose +Sel Marin | Glucose, $1H_2O$ | 55,000 g/L |
| | sels marins | 15,000 g/L |

(suite)

|  | Composé | C° milieu initial |
|---|---|---|
| Sels Minéraux | $MgSO_4, 7H_2O$ | 1,683 g/L |
|  | $K_2SO_4$ | 2,080 g/L |
|  | $KH_2PO_4$ | 4,000 g/L |
|  | $MnCl_2, 4H_2O$ | 10,800 mg/L |
|  | $ZnSO_4, 7H_2O$ | 1,080 mg/L |
|  | $CoCl_2, 6H_2O$ | 0,011 mg/L |
|  | $Na_2MoO_4, 2H_2O$ | 0,108 mg/L |
|  | $CuSO_4, 5H_2O$ | 0,720 mg/L |
|  | $NiSO_4, 6H_2O$ | 0,560 mg/L |
|  | $FeSO_4, 7H_2O$ | 40,000 mg/L |
| Vitamines | thiamine | 32,000 mg/L |
|  | Vitamine $B_{12}$ | 0,520 mg/L |
|  | Panthoténate | 10,800 mg/L |
| Azote | $(NH4)_2SO_4$ | 9,000 g/L |
| EDTA | $Na_2EDTA$ | 0,096 g/L |

Le mode de culture continu est ensuite démarré dès que la biomasse atteint 20 g/L dans le milieu. La solution d'alimentation apportée en continu est celle décrite dans le tableau 4 ci-dessous.

Tableau 4

| Composé | solution d'alimentation (g/L) |
|---|---|
| Glucose, $1H_2O$ | 110,000 |
| sels marins | 15,000 |
| $MgSO_4,7H_2O$ | 4,959 |
| $K_2SO_4$ | 6,129 |
| $KH_2PO_4$ | 11,786 |
| $MnCl_2\ 4H_2O$ | $3,182.10^{-2}$ |
| $ZnSO_4, 7H_2O$ | $3,182.10^{-2}$ |
| $CoCl_2, 6H_2O$ | $3,2.10^{-4}$ |
| $Na_2MoO_4, 2H_2O$ | $3,200.10^{-4}$ |
| $CuSO_4, 5H_2O$ | $2,121.10^{-2}$ |
| $NiSO_4, 6H_2O$ | $1,650.10^{-2}$ |
| $FeSO_4, 7H_2O$ | $1,179.10^{-1}$ |
| thiamine | $9,429.10^{-2}$ |
| Vitamine B12 | $1,530.10^{-3}$ |
| Panthoténate | $3,182.10^{-2}$ |
| $(NH_4)_2SO_4$ | 26,518 |
| EDTA | $4,092.10^{-1}$ |

Des essais préliminaires ont montré que le taux de dilution utilisé impacte directement la teneur en protéine de la biomasse (résultats non présentés). Pour cet exemple le taux de dilution utilisé est de 0,13 h$^{-1}$ ce qui correspond à la moitié du taux de croissance maximum de la souche dans les conditions de culture utilisées.

**Exemple 1.4 (comparatif)**

Des cultures de *Schizochytrium sp.* CCAP 4062/3 sont réalisées dans des fermenteurs comme essentiellement décrit dans l'Exemple 1.1, avec un milieu de culture complété avec du NaCl à hauteur respectivement de 27 g/L et 35,6 g/L pour le milieu pied fermenteur et le milieu d'ajout.

**Exemple 1.5 (invention)**

Les cultures de *Schizochytrium sp.* CCAP 4062/3 sont réalisées dans des fermenteurs comme essentiellement décrit dans l'Exemple 1.2, avec un milieu de culture complété avec du NaCl à hauteur respectivement de 27 g/L et 35,6 g/L pour le milieu pied fermenteur et le milieu d'ajout..

**Résultats**

|  | MS (g/l) | AA/MS (%) | MG/MS (%) |
|---|---|---|---|
| Soja (graines)* | - | 40,43 | 21,80 |
| Soja (tourteaux)* | - | 48,80 | 0,55 |
| Exemple 1.1 (comparatif) | 90,0 | 30,00 | 34,30 |
| Exemple 1.2 (invention) | 67,0 | 57,90 | 6,10 |
| Exemple 1.3 (invention) | 60,1 | 35,23 | 6,56 |
| Exemple 1.4 (comparatif) | 87,0 | 20,44 | 45,97 |
| Exemple 1.5 (invention) | 79,9 | 49,66 | 13,6 |
| MS : Matière sèche ; AA : Acides Aminés ; <br> * : Valeurs en % rapportées à 100 % de MS selon les valeurs données par l'USDA | | | |

On constate que les procédés de culture de l'état de la technique destinés à la production de matières grasses enrichies en acides gras polyinsaturés ne permettent pas d'obtenir des compositions à forte teneur en protéines (exprimée en % en poids d'acides aminés par rapport à la matière sèche).

**Exemple 2** - **Nutrition animale**

**Exemple 2.1 (Matériel et Méthodes)**

La souche *Aurantiochytrium Mangrovei* CC4062/5 a été cultivée dans les conditions de l'exemple 1.2. Ces conditions sont celles utilisées dans les exemples suivants sauf mention particulière.

**2.1.1** - **Composition de la biomasse** :

La biomasse microalgale a d'abord été analysée pour sa composition proximale ((humidité (Méthode interne EAU-H adaptée du Règlement CE g/100g 152/2009 du 27-01-2009 (103°C/4h) - SN), matières grasses par hydrolyse (Règlement CE 152/2009 du 27-01-2009 - procédé B - SN), protéines brutes estimées sur la base de la somme des concentrations en acides aminés totaux (Directive 98/64/CE, 3/09/99 - Norme NF EN ISO 13903), matières minérales (Règlement CE 152/2009 du 27-01-2009 - SN)), et énergie brute (NF EN 14918), fibres totales (AOAC 985.29 - SN), parois insolubles (selon la méthode de Carré et Brillouet (1989, Détermination of water insoluble cell walls in feeds: interlaboratory study. Journal Association of Official Analytical Chemists, 72, 463-467), calcium (NF EN ISO 6869 - mars 2002 - CT), phosphore (Règlement CE 152/2009 du 27-01-2009 - CT), chlorure (Méthode interne adaptée de ISO 1841-2 - juillet 1996 - SN), potassium (Méthode interne - ICP 05/07 - SN), sodium (Méthode interne - ICP 05/07 - SN) et concentrations en acides aminés totaux.

### 2.1.2 - Mesure de la digestibilité des acides aminés chez le coq caecactomisé:

En parallèle, cette microalgue est évaluée *in vivo* pour la mesure de la digestibilité de ses acides aminés chez le coq caecectomisé (Green et al., 1987). Les animaux (coqs adultes Isabrown) ont été alimentés par gavage humide avec une pâtée composée de l'un des ingrédients testés (microalgue testée ou un tourteau de soja contrôle) et complétée par de l'amidon de blé permettant d'obtenir un niveau de protéines de 18% correspondant au besoin des animaux. Douze coqs logés en cages individuelles ont été utilisés par traitement. Chaque animal a reçu en moyenne 171,2 $\pm$ 14,5 g de pâtée après un jeûne de 24h. Les excrétas collectés jusqu'à 48h après gavage ont été regroupés par groupe de 4 coqs de telle façon que 3 excrétas par traitement soient lyophilisés. Ils ont ensuite été laissés à température ambiante pour une phase de reprise en eau (48h) afin de stabiliser le contenu en humidité puis pesés, broyés (0,5 mm) et analysés par la méthode Terpstra (Terpstra et de Hart, 1974) pour leur teneur en azote non urique et pour leurs concentrations en acides aminés totaux (JEOL AMINOTAC JLC 500/V). Les pertes endogènes en acides aminés ont précédemment été déterminées en évaluant un régime protéiprive (c'est-à-dire sans aucun apport en protéines) sur la base du même schéma expérimental. Les résultats corrigés des pertes endogènes basales sont donc exprimés en coefficient de digestibilité iléale standardisée des acides aminés, calculés et soumis à l'analyse statistique comme suit :

$$DIS(X)_F = \left[ \frac{X_F * I_D * L_F - X_E * Q_E + \dfrac{X_{END} * I_D}{10}}{X_F * I_D * L_F} \right] * 1000$$

avec DIS $(X)_F$ : coefficient de digestibilité iléale standardisée (%) de l'acide aminé X de la matière première F ; $X_F$ : concentration (g/100g) de l'acide aminé dans la matière première ; $X_E$ : concentration (g/100g) de l'acide aminé dans les excrétas ; $I_D$: quantité d'aliment consommée (g) ; $Q_E$ : quantité excrétée (g) ; $L_F$: taux d'incorporation de la matière première dans l'aliment ; $X_{END}$ : pertes endogènes de l'acide aminé X (g/100g).

Les résultats expérimentaux sont d'abord analysés pour l'effet ingrédient selon une procédure ANOVA (XLSTAT 2010.4.02 © Addinsoft 1995-2010), d'après le modèle suivant à 95% d'intervalle de confiance:

$$Yi = \mu + ai$$

avec :

Y = paramètre
$\mu$ = moyenne
ai = effet de l'ingrédient

Pour chaque différences significatives, les moyennes sont analysées par un pair-wise Fisher's LSD test.

### Exemple 2.2 : Composition de la souche CC4062/5 cultivée durant 49h.

La souche CC4062/5 a été cultivée comme mentionné ci-dessus. La culture a été stoppée à 49h, donnant lieu à une production de biomasse microalgale de quelques kilos et dans l'objectif d'organiser un test préliminaire de digestibilité chez l'animal. Cette biomasse a été séchée sur sécheur à cylindre et se présente sous la forme de paillettes. Elle est identifiée « souche n°4, culture 49h ».

Les résultats de composition biochimique de la « microalgue, souche 4, culture 49h » sont présentés dans le tableau 5 ci-dessous et exprimés à la fois en brut et corrigés de la teneur en matière sèche (MS).

**Tableau 5 (essai 14ALG058):** Composition de la microalgue (souche n°4, culture 49h) testée vs un tourteau de soja témoin

| | Concentrations (% brut) | | Concentrations (% matière sèche) | |
|---|---|---|---|---|
| | μA | Ttx soja | μA | Ttx soja |
| Matière Sèche, % | 94,8 | 89,5 | - | - |

(suite)

| | Concentrations (% brut) | | Concentrations (% matière sèche) | |
|---|---|---|---|---|
| | µA | Ttx soja | µA | Ttx soja |
| Humidité, % | 5,2 | 10,5 | - | - |
| Somme des acides aminés | 50,8 | 46,9 | 53,4 | 52,4 |
| Matières Grasses | 10,6 | 5,0 | 11,2 | 5,6 |
| Cellulose brute | n/d | 4,2 | n/d | 4,7 |
| Matières Minérales | 7,3 | 5,8 | 7,7 | 6,5 |
| Amidon | <1,0 | n/a | <1,1 | n/a |
| Sucres totaux | 1,3 | n/d | 1,4 | n/d |
| ADF | n/d | 3,7 | n/d | 4,1 |
| NDF | n/d | 7,2 | n/d | 8,0 |
| Fibres totales (TDF, total dietary fiber) | 16,5 | n/d | 17,4 | n/d |
| Parois insolubles (WICW, water insoluble cell wall) | 4,9 | n/d | 5,2 | n/d |
| Calcium | 0,118 | 0,371 | 0,124 | 0,414 |
| Phosphore | 1,448 | 0,598 | 1,527 | 0,668 |
| Chlorure (Cl-) | 0,150 | n/d | 0,158 | n/d |
| Potassium | 1,983 | n/d | 2,092 | n/d |
| Sodium | 0,068 | n/d | 0,072 | n/d |
| | (kcal/kg) | | (kcal/kg MS) | |
| Energie brute | 4870 | 4486 | 5137 | 5012 |

*nld: non déterminé*
*nla: non applicable*
µA : microalgue ; Ttx : Tourteau de Soja

Ces résultats montrent que la microalgue présente une composition en matières azotées totales (somme des acides aminés) et matières minérales légèrement supérieure à celles du tourteau de soja témoin. La teneur en minéraux de la biomasse testée est assimilable aux concentrations les plus faibles publiées et allant de 7 à 43 % MS selon les familles, genres et espèces de microalgues. Les teneurs en phosphore et calcium sont inversement proportionnées avec 2,3 fois plus de phosphore et 3,3 fois moins de calcium dans la microalgue par rapport au tourteau de soja témoin. La « souche 4, culture 49h » testée ne contient pas de réserves d'amidon et sa concentration en sucres totaux reste faible (1,4 % MS) comparativement aux matières premières classiquement utilisées en nutrition animale. A contrario, sa teneur en matières grasses est deux fois plus importante que celle du tourteau de soja (11,2 vs 5,6 % MS) et son énergie brute supérieure de 125 kcal/kg MS.

Les parois des microalgues présentent des compositions et structures différentes de celles des matières premières d'origine végétales, et rendent les analyses de fibres classiquement utilisées non adaptées. Aussi, l'analyse des fibres totales (TDF) (Prosky *et al.,* 1988) présente l'avantage d'être moins restrictive et la « microalgue, souche 4, culture 49h » présente un pourcentage de fibres totale de 17,4 % MS (ce qui à titre de comparaison est de l'ordre de ce que l'on analyse dans le grain d'orge ou de seigle) avec un résidu en paroi végétale insoluble (WICW) mesuré à 5,2 % de MS.

Les concentrations en protéines totales rapportées dans les tableaux 4 à 8 sont issues de la somme des acides aminés eux-mêmes dosés selon la méthode décrite précédemment. La somme des acides aminés -comme estimateur des protéines totales de la « microalgue, souche 4, culture 49h »- correspond à 53,4 % MS, avec plus précisément 21,5 % MS d'acides aminés essentiels et 32,0 % MS d'acides aminés non essentiels. Le tableau 6 présente les concentrations en acides aminés analysés dans la microalgue et le tourteau de soja et exprimées en brut ou rapportées à la somme des acides aminés.

Les résultats reflètent un profil en acides aminés de la microalgue relativement équilibré, ce qui rejoint des travaux comparant des profils en acides aminés de différentes microalgues à ceux de sources protéiques dites conventionnelles (Becker, 2007).

De façon générale, les concentrations en acides aminés essentiels de la microalgue sont légèrement supérieures à celles mesurées dans le tourteau de soja (respectivement 21,5 % MS et 24,0 % MS).

**Tableau 6 (essai 14ALG058)** : Concentrations en azote total et en acides aminés (% brut), concentrations en acides aminés rapportées à la somme des acides aminés (% somme AA), coefficients de digestibilité iléale standardisée des acides aminés (%) mesurés chez le coq caecectomisé et concentrations en acides aminés digestibles (% brut) de la microalgue testée vs un tourteau de soja témoin.

| | [AA] (% brut) | | AA / somme AA (%) | | Digestibilité iléale standardisée des AA (%) | | | [AA] digestibles (% brut) | |
|---|---|---|---|---|---|---|---|---|---|
| | μA | Ttx soja | μA | Ttx soja | μA | Ttx soja | P-value | μA | Ttx soja |
| Matière Sèche | **94,8** | **89,5** | - | - | - | - | - | - | - |
| Azote total | 10,6 | 7,8 | - | - | 80,1 ± 0,3 | 89,6 ± 0,7 | **< 0,0001** | 8,49 | 6,97 |
| Somme AA | 50,8 | 46,9 | - | - | 78,4 ± 1,0 | 88,5 ± 1,0 | **0,001** | 39,79 | 41,5 3 |
| **AA essentiels** | **20,4** | **21,5** | | | | | | | |
| Méthionine | 0,85 | 0,64 | 1,67 | 1,36 | 85,6 ± 1,3 | 90,9 ± 1,6 | **0,023** | 0,73 | 0,58 |
| Lysine | 2,30 | 2,87 | 4,53 | 6,12 | 84,9 ± 1,3 | 87,6 ± 0,8 | 0,068 | 1,95 | 2,51 |
| Thréonine | 1,71 | 1,88 | 3,37 | 4,01 | 80,7 ± 1,8 | 86,0 ± 1,7 | **0,038** | 1,38 | 1,62 |
| Tryptophane | 0,55 | 0,67 | 1,08 | 1,43 | 82,9 ± 1,7 | 90,3 ± 1,3 | **0,009** | 0,46 | 0,61 |
| Arginine | 6,83 | 3,42 | 13,44 | 7,29 | 61,0 ± 0,8 | 93,2 ± 0,8 | **< 0,0001** | 4,17 | 3,19 |
| Isoleucine | 1,48 | 2,30 | 2,91 | 4,90 | 85,4 ± 1,2 | 88,7 ± 1,2 | **0,048** | 1,26 | 2,04 |
| Leucine | 2,52 | 3,69 | 4,96 | 7,87 | 86,7 ± 1,1 | 88,5 ± 1,2 | 0,182 | 2,18 | 3,27 |
| Valine | 2,01 | 2,35 | 3,96 | 5,01 | 87,5 ± 1,6 | 89,0 ± 1,5 | 0,389 | 1,76 | 2,09 |
| Histidine | 0,66 | 1,18 | 1,30 | 2,52 | 88,7 ± 0,6 | 89,9 ± 1,4 | 0,327 | 0,59 | 1,06 |
| Phénylalanine | 1,52 | 2,48 | 2,99 | 5,29 | 84,6 ± 1,5 | 89,8 ± 1,0 | **0,014** | 1,29 | 2,23 |
| **AA non essentiels** | **30,3** | **25,5** | | | | | | | |
| Cystine | 0,55 | 0,63 | 1,08 | 1,34 | 60,9 ± 3,7 | 79,7 ± 2,0 | **0,003** | 0,34 | 0,50 |
| Serine | 1,87 | 2,52 | 3,68 | 5,37 | 76,7 ± 1,6 | 88,5 ± 1,3 | **0,001** | 1,43 | 2,23 |
| Proline | 1,07 | 2,14 | 2,11 | 4,56 | 84,3 ± 2,1 | 87,9 ± 1,3 | 0,107 | 0,90 | 1,88 |

(suite)

| | [AA] (% brut) | | AA / somme AA (%) | | Digestibilité iléale standardisée des AA (%) | | | [AA] digestibles (% brut) | |
|---|---|---|---|---|---|---|---|---|---|
| | $\mu$A | Ttx soja | $\mu$A | Ttx soja | $\mu$A | Ttx soja | P-value | $\mu$A | Ttx soja |
| Alanine | 2,17 | 2,07 | 4,27 | 4,41 | 84,9 ± 1,1 | 84,9 ± 1,1 | 0,992 | 1,84 | 1,76 |
| Ac. Aspartique | 3,57 | 5,49 | 7,03 | 11,71 | n/d | 85,9 ± 0,6 | n/d | n/d | 4,71 |
| Ac. Glutamique | 18,10 | 8,81 | 35,63 | 18,78 | 73,4 ± 0,7 | 90,4 ± 0,7 | **0,0001** | 13,29 | 7,96 |
| Glycine | 1,81 | 2,01 | 3,56 | 4,29 | 78,3 ± 2,3 | 84,3 ± 1,6 | **0,036** | 1,42 | 1,69 |
| Tyrosine | 1,18 | 1,78 | 2,32 | 3,80 | 84,2 ± 1,7 | 89,5 ± 0,6 | **0,015** | 0,99 | 1,59 |
| $\mu$A : microalgue ; Ttx soja : Tourteaux de soja ; [AA] : Concentration en acides aminés | | | | | | | | | |

### Exemple 2.3 : Mesure de la digestibilité des acides aminés de la biomasse (souche CC4062/5 cultivée pendant 49h)

Les résultats de digestibilité iléale standardisée (DIS) des acides aminés mesurés chez le coq caecectomisé sont présentés dans le tableau 6 ainsi que les concentrations en acides aminés digestibles. Les digestibilités de l'azote et de la somme des acides aminés de la microalgue sont respectivement de 80,1 ± 0,3 % et de 78,4 ± 1,0 % soit 9,5 et 10,1 points de moins que le témoin tourteau de soja. Les SID arginine et cystine de la microalgue présentent les résultats les plus faibles, respectivement 61,0 ± 0,8 % et 60,9 ± 3,7 %. De façon générale, les coefficients de digestibilité des acides aminés essentiels de la microalgue vont de 80,7 ± 1,8 % (DIS thréonine) à 88,7 ± 0,6 % (DIS histidine). La digestibilité de la lysine est mesurée à 85,6 ± 1,3 %. Ces coefficients sont de l'ordre de 1,2 (SID histidine) à 7,4 (SID tryptophane) points inférieurs à ceux mesurés pour le tourteau de soja contrôle. Cependant, ils reflètent des coefficients de digestibilité élevés, permettant de considérer la microalgue évaluée comme une source protéique de bonne qualité (i.e. ils sont significativement plus élevés que les valeurs de digestibilité des protéines de microalgues rapportées dans la littérature de l'ordre de 55 à 77% (Henman *et al.,* 2012).

### Exemple 2.4 : Composition de la souche CC4062/5 cultivée pendant 22h

La souche CC4062/5 a été cultivée dans les mêmes conditions mais pendant une durée de 22h. Elle est identifiée « souche n°4, culture 22h ».

Comme précédemment, cette biomasse a été séchée sur sécheur à cylindre et se présente sous la forme physique de paillettes.

Elle a été analysée pour sa composition en proximales, énergie brute, fibres totales, parois insolubles, calcium, phosphore, chlorure, potassium, sodium et concentrations en acides aminés totaux.

La composition biochimique de cette « microalgue, souche 4, culture 22h » est présentée dans le tableau 7. Les résultats sont exprimés à la fois en brut et corrigés de la teneur en matière sèche (MS).

La « microalgue, souche 4, culture 22h » présente une composition en matières azotées totales (sommes des acides aminés) de 2,3 et 1,3 points supérieure à celles du tourteau de soja témoin et de la « microalgue, souche n°4, culture 49h ».

Sa teneur en matières minérales augmente par rapport à une culture plus longue mais reste à un niveau de concentration qui se situe dans la gamme inférieure des valeurs publiées dans la littérature (Skrede et *al*., 2011).

Le ratio phosphore/calcium est déséquilibré. Les teneurs en phosphore et calcium sont inversement proportionnées avec 3,6 fois plus de phosphore et 3,6 fois moins de calcium dans la microalgue par rapport au tourteau de soja témoin. La souche n°4 optimisée (culture 22h) contient 1,5 fois plus de phosphore total que lors d'une culture de 49h. La teneur en matières grasses est supérieure à celle du tourteau de soja (8 % vs 5,6 % MS), et les résultats confirment que ni l'amidon, ni les sucres totaux ne représentent une forme de stockage d'énergie chez cette souche n°4. L'énergie brute est mesurée à 4641 kcal/kg MS, soit 371 kcal/kg MS de moins vs le contrôle tourteau de soja.

Les fibres totales (TDF) de la « microalgue, souche 4, culture 22h » représentent 12,7 % de la MS avec un résidu en parois végétales insolubles (WICW) mesuré à 2,1 % MS. En parallèle, le résidu déduit comme suit : R (%) = 100 - MM (%) - MAT (%) - MG (%) - amidon (%) - sucres (%) indique que la teneur en TDF est inférieure de 15,1 points à celle du R ainsi estimé à 27,8 % MS. Ces observations rejoignent d'autres travaux (Lieve et *al*., 2012) rapportant de l'ordre de 20 à 30% de la quantité de la biomasse sèche non expliquée par la somme des teneurs en matières minérales, lipides, protéines et hydrates de carbone.

La « microalgue, souche 4, culture 22h » présentant 2 points de plus de matières grasse que le tourteau de soja pris en comparaison (tableau 7).

**Tableau 7 (essai 14ALG065):** Composition de la microalgue (souche n°4, culture 22h) testée vs un tourteau de soja témoin

| | Concentrations en AA (% brut) | | Concentrations en AA (% matière sèche) | |
|---|---|---|---|---|
| | μA | Ttx soja | μA | Ttx soja |
| Matière Sèche | 94,6 | 89,5 | - | - |
| Humidité | 5,4 | 10,5 | - | - |
| Somme des acides aminés | 51,7 | 46,9 | 54,7 | 52,4 |
| Matières Grasses | 7,6 | 5,0 | 8,0 | 5,6 |
| Cellulose brute | n/d | 4,2 | n/d | 4,7 |
| Matières Minérales | 9,0 | 5,8 | 9,5 | 6,5 |
| Amidon | <0,5 | n/a | <0,5 | <0,6 |
| Sucres totaux | <0,5 | n/d | <0,5 | <0,6 |
| ADF | n/d | 3,7 | n/d | 4,1 |
| NDF | n/d | 7,2 | n/d | 8,0 |
| Fibres totales (TDF, total dietary fiber) | 12,0 | n/d | 12,7 | n/d |
| Parois insolubles (WICW, water insoluble cell wall) | 2,0 | n/d | 2,1 | n/d |
| Calcium | 0,109 | 0,371 | 0,115 | 0,415 |
| Phosphore | 2,279 | 0,598 | 2,410 | 0,668 |
| Chlorure (Cl-) | 0,160 | n/d | 0,169 | n/d |
| Potassium | 2,238 | n/d | 2,366 | n/d |
| Sodium | 0,161 | n/d | 0,170 | n/d |
| | (kcal/kg) | | (kcal/kg MS) | |
| Energie brute | 4390 | 4486 | 4641 | 5012 |
| *n/d: non déterminé ; n/a: non applicable ;* μA : microalgue ; Ttx soja : Tourteaux de soja ; | | | | |

La somme des acides aminés s'élève à 54,7 % de la MS, avec plus précisément 23,9 % MS d'acides aminés essentiels et 30,8 % MS d'acides aminés non essentiels. Le tableau 8 présente les concentrations en acides aminés analysés dans la microalgue et le tourteau de soja et exprimées en brut ou rapportées à la somme des acides aminés. Les résultats reflètent un profil en acides aminés de la microalgue relativement équilibré par rapport au tourteau de soja contrôle. L'acide glutamique, l'arginine et l'acide aspartique de la « microalgue, souche 4, culture 22h » sont présents en plus forte proportion avec des valeurs (% somme AA) respectivement de 26,75, 9,48 et 8,26 %. La teneur en lysine (% somme AA) est de 5,94 % en référence à celle du tourteau de soja témoin (6,12 %). L'arginine, la méthionine et dans une moindre mesure la thréonine contribuent plus fortement à la protéine de microalgue testée qu'à celle du tourteau de soja (9,48 vs 7,29 %, 2,13 vs 1,36 % et 4,27 vs 4,01 % respectivement). La microalgue cultivée durant 22h présente 2,4 points d'acides essentiels de plus dans le cas d'une culture de 49h soit la même teneur que celle du tourteau de soja contrôle. A l'exception de l'arginine, les acides aminés essentiels sont présents en proportion plus importante de la somme des acides aminés.

La « microalgue, souche 4, culture 22h » présente des teneurs en méthionine, arginine et thréonine supérieures à celles du soja tandis que les teneurs en lysine et valine sont équivalentes voire un peu supérieures dans la biomasse microalgale.

**Exemple 2.5 : Mesure de la digestibilité des acides aminés de la biomasse (souche CC4062/5 cultivée pendant 22h)**

La mesure de la digestibilité de ses acides aminés chez le coq caecectomisé (Green et *al*., 1987) de façon identique à celle décrite précédemment (cf. Exemple 2.3).

Les résultats de digestibilité iléale standardisée (DIS) des acides aminés de la « microalgue souche 4, culture 22h » mesurés chez le coq caecectomisé sont présentés dans le tableau 8 ainsi que les concentrations en acides aminés digestibles. Les digestibilités de l'azote et de la somme des acides aminés de la microalgue sont respectivement de 85,7 ± 0,7 % et de 87,1 ± 0,4 % soit 3,2 (P = 0,008) et 0,9 (P = 0,190) points de moins que le témoin tourteau de soja. Par rapport à la culture de 49h, la digestibilité de la somme des acides aminés de cette production de « microalgue, souche 4, culture 22h » est significativement augmentée de 8,7 points. Les concentrations en lysine, méthionine, arginine, thréonine, valine digestibles sont supérieures à celles du tourteau de soja contrôle. Elles reflètent une bonne qualité de la protéine de la microalgue testée, supérieure ou équivalente à celle d'un tourteau de soja.

Les résultats de mesure de la digestibilité de ses acides aminés chez le coq, montrent que la « microalgue, souche n°4, culture 22h » est une source protéique de bonne qualité. La digestibilité des protéines (somme des acides aminés) est similaire à celle d'un tourteau de soja qui est la source protéique la plus utilisée dans le monde pour l'alimentation des monogastriques.

De plus, le procédé de séchage utilisé n'apparait pas comme un facteur dénaturant la qualité de la protéine étant donné les coefficients de digestibilité élevés mesurés pour la lysine.

Il est important de noter que la qualité de la protéine s'est significativement améliorée lorsque l'on compare les résultats de la « microalgue, souche n°4, culture 49h » à ceux de la « microalgue, souche n°4, culture 22h ». Notamment, la digestibilité des protéines augmente d'environ 9 points.

**Tableau 8 (essai 14ALG065):** Concentrations en azote total et en acides aminés (% brut), concentrations en acides aminés rapportées à la somme des acides aminés (% somme AA), coefficients de digestibilité iléale standardisée des acides aminés (%) mesurés chez le coq caecectomisé et concentrations en acides aminés digestibles (% brut) de la microalgue testée vs un tourteau de soja témoin.

| | [AA] (% brut) | | AA (%) | | Digestibilité iléale standardisée des AA (%) | | | [AA] digestibles (% brut) | |
|---|---|---|---|---|---|---|---|---|---|
| | µA | Ttx soja | µA | Ttx soja | µA | Ttx soja | *P*-value | µA | Ttx soja |
| Matière Sèche | 94,6 | 89,5 | - | - | - | - | - | - | - |
| Azote total | 11,5 | 7,8 | - | - | 85,7± 0,7 | 88,9± 0,5 | **0,008** | 9,86 | 6,92 |
| Somme AA | 51,7 | 46,9 | - | - | 87,1± 0,4 | 88,0± 0,7 | 0,190 | 45,06 | 41,2 |
| **AA essentiels** | **22,6** | **21,5** | | | | | | | |
| Méthionine | 1,10 | 0,64 | 2,13 | 1,36 | 90,0 ± 0,4 | 91,5 ± 0,3 | **0,015** | 0,99 | 0,59 |
| Lysine | 3,07 | 2,87 | 5,94 | 6,12 | 87,9 ± 0,2 | 85,6 ± 0,3 | **0,0001** | 2,70 | 2,46 |
| Thréonine | 2,21 | 1,88 | 4,27 | 4,01 | 87,3 ± 0,3 | 85,2 ± 1,0 | **0,045** | 1,93 | 1,60 |
| Tryptophane | 0,68 | 0,67 | 1,32 | 1,43 | 86,0 ± 0,2 | 89,3 ± 1,1 | **0,013** | 0,59 | 0,60 |
| Arginine | 4,90 | 3,42 | 9,48 | 7,29 | 79,9 ± 1,1 | 93,0 ± 0,5 | **< 0,0001** | 3,92 | 3,18 |

(suite)

| | [AA] (% brut) | | AA (%) | | Digestibilité iléale standardisée des AA (%) | | | [AA] digestibles (% brut) | |
|---|---|---|---|---|---|---|---|---|---|
| | μA | Ttx soja | μA | Ttx soja | μA | Ttx soja | P-value | μA | Ttx soja |
| Isoleucine | 1,98 | 2,30 | 3,83 | 4,90 | 88,2 ± 0,3 | 88,5 ± 0,5 | 0,526 | 1,75 | 2,04 |
| Leucine | 3,31 | 3,69 | 6,40 | 7,87 | 89,0 ± 0,2 | 88,5 ± 0,7 | 0,341 | 2,95 | 3,27 |
| Valine | 2,57 | 2,35 | 4,97 | 5,01 | 87,1 ± 0,2 | 85,6 ± 1,2 | 0,153 | 2,24 | 2,01 |
| Histidine | 0,82 | 1,18 | 1,59 | 2,52 | 88,7 ± 2,1 | 89,6 ± 0,7 | 0,578 | 0,73 | 1,06 |
| Phénylalanine | 1,96 | 2,48 | 3,79 | 5,29 | 87,4 ± 0,4 | 89,8 ± 0,7 | **0,012** | 1,71 | 2,23 |
| **AA non essentiels** | **29,1** | **25,5** | | | | | | | |
| Cystine | 0,65 | 0,63 | 1,26 | 1,34 | 69,4 ± 1,2 | 79,1 ± 2,3 | **0,006** | 0,45 | 0,50 |
| Serine | 2,09 | 2,52 | 4,04 | 5,37 | 83,0 ± 0,5 | 87,7 ± 1,0 | **0,004** | 1,73 | 2,21 |
| Proline | 1,66 | 2,14 | 3,21 | 4,56 | 87,9 ± 0,8 | 87,2 ± 1,2 | 0,519 | 1,46 | 1,87 |
| Alanine | 2,80 | 2,07 | 5,42 | 4,41 | 88,8 ± 0,3 | 84,7 ± 1,0 | **0,005** | 2,49 | 1,75 |
| Ac. Aspartique | 4,27 | 5,49 | 8,26 | 11,71 | n/d | 85,7 ± 0,8 | n/d | n/d | 4,71 |
| Ac. Glutamique | 13,83 | 8,81 | 26,7 5 | 18,78 | 84,8 ± 0,7 | 90,5 ± 0,6 | **0,001** | 11,72 | 7,97 |
| Glycine | 2,34 | 2,01 | 4,53 | 4,29 | 81,8 ± 0,9 | 83,3 ± 0,5 | 0,125 | 1,92 | 1,67 |
| Tyrosine | 1,48 | 1,78 | 2,86 | 3,80 | 86,7 ± 0,1 | 89,4 ± 0,9 | **0,016** | 1,28 | 1,59 |

*n/d: non déterminé ;* μA : microalgue ; μA : microalgue ; Ttx soja : Tourteaux de soja ; [AA] : Concentration en acides aminés

**Exemple 2.6 : détermination de la valeur d'Energie Métabolisable Apparente (EMA) et de l'Energie Métabolisable Apparente corrigée du bilan azoté (EMAn) de la souche CC4062/5 cultivée pendant 22h.**

La mesure de la valeur d'Energie Métabolisable Apparente (EMA) de la microalgue « microalgue, souche 4, culture 22h » a été faite chez le poulet à 3 semaines d'âge (Bourdillon *et al.,* 1990). La méthode dite de substitution a été appliquée à partir d'un régime témoin composé d'une base maïs-soja et d'un premix. De même que les autres matières premières, la microalgue, broyée sur une grille de 3mm a été incorporée à des taux croissants de 4, 8, 12, 16 et 20% en substitution au mélange maïs-soja et en gardant constant le premix dans les régimes.

Le tableau 9 présente la formule de l'aliment maïs-soja contrôle ainsi que ses spécifications (Protéine brute = 18%, Energie Métabolisable Apparente = 3200 kcal/kg). Les aliments substitués par la microalgue sont équilibrés (notamment en leur ratio protéine sur énergie) au mieux et sur la base d'hypothèses EMA, acides aminés digestibles et phosphore biodisponible prises pour la microalgue.

**Tableau 9 (essai 14ALG069):** Formule de l'aliment expérimental complémentaire utilisé

|  | R1 |
| --- | --- |
|  | **Basal Maïs-Soja** |
| Maïs | 67,673 |
| Tourteau de soja 48% | 19,742 |
| Gluten de maïs 60 | 4,766 |
| Huile de soja | 3,139 |
| Premix | 4,680 |
| Microalgue | 0 |
|  |  |
| Protéine brute | 18 |
| Matières Grasses | 6,201 |
| Cellulose brute | 3,029 |
| Matières Minérales | 2,013 |
| Lysine | 0,80 |
| Methionine | 0,33 |
| Met+Cys | 0,664 |
| Threonine | 0,686 |
| Tryptophane | 0,173 |
| Arginine | 1,066 |
| Calcium | 0,1 |
| Phosphore total | 0,333 |
| Phosphore disponible | 0,078 |
| Sodium | 0,092 |
| EM, kcal/kg | 3200 |

Régime 1: basal maïs-soja (95,32%) + CMV, complément minéraux vitamines (4,68%)
Régime 2: basal maïs-soja (91,32%) + CMV (4,68%) + microalgue (4%)
Régime 3: basal maïs-soja (87,32%) + CMV (4,68%) + microalgue (8%)
Régime 4: basal maïs-soja (83,32%) + CMV (4,68%) + microalgue (12%)
Régime 5: basal maïs-soja (79,32%) + CMV (4,68%) + microalgue (16%)
Régime 6: basal maïs-soja (75,32%) + CMV (4,68%) + microalgue (20%)

Ce gradient d'incorporation permet d'évaluer la valeur EMA de la microalgue par régression linéaire simple. Le dispositif expérimental est décrit en Figure 1. Les valeurs de matière sèche des régimes ont été déterminées après broyage et sur aliments granulés au début et à la fin de la période de bilan. Enfin, les aliments complets et les fèces ont été analysés individuellement pour leur teneur en énergie brute (EB) par calorimétrie.

L'EMAn des aliments est calculé de la façon suivante :

$$EMA\,(kcal\,/\,kg\ MS) = \frac{\left(I \times EBi - E \times EBf\right)}{I}$$

$$EMAn \ (kcal \, / \, kg \, MS) = EMA - 8.22 \, \frac{(GP \times 0.18) \big/ 6.25}{I}$$

avec I : quantité d'aliment ingéré (kg MS) ; E : quantité d'excrétas (kg) ; EBi et EBf : énergie brute de l'ingéré (kcal/kg MS) et des fèces (kcal/kg); GP : gain de poids pendant la période de bilan. Les valeurs d'EMA ont dans un premier temps été corrigées par le bilan azoté sur la base de 18% de protéines contenu dans les tissus des animaux et en utilisant le facteur de 8,22 kcal/kg N (Hill et Anderson, 1958). En parallèle, les calculs permettant la correction du bilan azoté ont aussi été faits sur la base du dosage de l'azote total dans les excrétas. Les deux méthodes donnant des résultats non différents, c'est cette dernière méthode qui est considérée dans ce rapport. Les valeurs aberrantes ($\pm$ 2,5 écarts types de la moyenne) ont été éliminées avant traitement par analyse de variance (ANOVA, SAS 9.1.3 [©] 2002-2003 by SAS Institute Inc., Cary, NC, USA) selon un schéma factoriel. La digestibilité de la matière sèche (dMS), le coefficient de digestibilité apparente de l'azote (CUDa azote), du phosphore (CUDa phosphore), du calcium (CUDa calcium) et de la matière grasse (CUDa MG) sont calculés de la même façon pour chacun des régimes.

Ces résultats ont été analysés par régression selon la procédure de Régression Linéaire ((XLSTAT 2010.4.02 [©] Addinsoft 1995-2010), d'après le modèle suivant :

$$Yi = \beta 0 + \beta j X i j + \varepsilon i$$

Avec :

Yi = valeur observée pour la variable dépendante pour l'observation i,

$\beta$0 = intercept

$\beta$j = pente de la droite de régression

Xij = valeur prise par la variable j pour l'observation i (% d'incorporation de la microalgue),

$\varepsilon$i = erreur du modèle.

La valeur du paramètre pour une incorporation de 100% de la microalgue a été extrapolée à partir du modèle de prédiction, permettant ainsi d'estimer l'EMA et EMAn et le coefficient de digestibilité apparente du phosphore de la microalgue.

Le tableau 10 présente les résultats de digestibilité de la matière sèche et de l'énergie métabolisable apparente corrigée du bilan azoté (EMAn) de l'aliment maïs-soja contrôle ainsi que des régimes substitués R2 à R6. Les coefficients de digestibilité apparente de l'azote (CUDa azote), du phosphore (CUDa phosphore) et du calcium (CUDa calcium) et de la matière grasse (CUDa MG) ont été estimés par la même méthode.

**Tableau 10 (essai 14ALG069):** Digestibilité de la Matière Sèche, de l'énergie, et coefficient d'utilisation digestive apparente d'aliments expérimentaux respectivement substitués à 0, 4, 8, 12, 16 et 20% de la microalgue testée (souche n°4, culture 22h) et mesurés chez le poulet de 23 jours d'âge

| Régimes | Aliments expérimentaux | | | | | | P-value |
|---|---|---|---|---|---|---|---|
| | R1 | R2 | R3 | R4 | R5 | R6 | |
| | B M-S | B M-S + 4% µA | B M-S + 8% µA | B M-S + 12% µA | B M-S + 16% µA | B M-S + 20% µA | |
| dMS (%)[1] | 75,10 $\pm$ 1,20a | 74,41 $\pm$ 1,09ab | 73,53 $\pm$ 1,64b | 71,64 $\pm$ 1,43c | 70,17 $\pm$ 1,22d | 69,56 $\pm$ 2,05d | < 0,0001 |
| EMAn (kcal/kg MS)[2] | 3398,0 $\pm$ 53a | 3379,4 $\pm$ 42a | 3331,8 $\pm$ 64b | 3273,6 $\pm$ 61c | 3218,9 $\pm$ 53d | 3171,4 $\pm$ 72e | < 0,0001 |
| EB/EMAn (%)[3] | 78,35 $\pm$ 1,34a | 78,05 $\pm$ 1,25a | 77,71 $\pm$ 1,72a | 76,19 $\pm$ 1,56b | 75,00 $\pm$ 1,56c | 74,72 $\pm$ 1,84c | < 0,0001 |

(suite)

| Régimes | Aliments expérimentaux | | | | | | P-value |
|---|---|---|---|---|---|---|---|
| | R1 | R2 | R3 | R4 | R5 | R6 | |
| | B M-S | B M-S + 4% µA | B M-S + 8% µA | B M-S + 12% µA | B M-S + 16% µA | B M-S + 20% µA | |
| CUDa azote (%)[4] | 61,79 ± 3,75a | 60,24 ± 2,44ab | 58,80 ± 4,87b | 56,48 ± 3,15c | 53,97 ± 2,26d | 53,90 ± 3,44d | < 0,0001 |
| CUDa phosphore (%)[5] | 38,00 ± 4,67a | 40,78 ±4,36b | 41,42 ± 4,87b | 36,96 ± 4,89a | 37,77 ± 3,38a | 37,28 ± 3,83a | 0,004 |
| CUDa calcium (%)[6] | 26,84 ± 5,41a | 34,32 ± 5,85b | 37,86 ± 5,91ab | 34,90 ± 4,61bc | 36,74 ± 4,55bcd | 38,94 ± 5,01d | < 0,0001 |
| CUDa MG (%)[7] | 85,88 ± 4,2ab | 87,17 ± 1,88ac | 88,59 ± 2,53c | 85,93 ± 3,33a | 83,97 ± 3,37bd | 83,86 ± 3,14d | < 0,0001 |

B M-S : Basal Maïs-Soja ; µA : microalgue [1] : Digestibilité de la Matière Sèche (%) ; [2] : Energie Métabolisable Apparente corrigée du bilan azoté (AMEn) ; [3] : Energie brute rapportée à l'AMEn (%) ; [4] : Coefficient de digestibilité apparente de l'azote ; [5] : Coefficient de digestibilité apparente du phosphore ; [6] : Coefficient de digestibilité apparente du calcium, [7] : Coefficient de digestibilité apparente de la Matière Grasse (%)

La digestibilité de la matière sèche des différents régimes est fortement corrélée à leurs valeurs d'EMAn. L'EMAn du régime maïs-soja R1 est mesurée à 3398 ± 53 kcal/kg MS. Les résultats montrent que la valeur EMAn du régime R2 est équivalente à celle du contrôle maïs-soja et reflètent que la substitution à hauteur de 4% par la microalgue n'impacte pas la digestibilité de l'énergie du régime. A contrario, et à partir d'une substitution supérieure ou égale à 8% de microalgue, l'EMAn ainsi que le CUDa azote diminuent de façon linéaire avec les pourcentages croissants de substitution (P < 0,0001).

L'addition de 4 à 16% de micro algue montre une diminution de l'indice de consommation (IC) sur la période 13-23 jours. Il est à noter aussi que les poulets tendent à moins consommer (à l'exception du R2) pour cependant un gain de poids similaire quel que soit le pourcentage d'incorporation de la microalgue et supérieur à celui mesuré pour le régime contrôle maïs-soja (Tableau 11).

**Tableau 11 (essai 14ALG069):** Gain de poids, consommation et indice de consommation des animaux sur la période 13 à 23 jours alimentés des régimes respectivement substitués à 0, 4, 8, 12, 16 et 20% de la microalgue testée (souche n°4, culture 22h).

| | Aliments expérimentaux | | | | | |
|---|---|---|---|---|---|---|
| | R1 | R2 | R3 | R4 | R5 | R6 |
| | B M-S | B M-S +4% µA | B M-S +8% µA | B M-S +12% µA | B M-S +16% µA | B M-S +20% µA |
| Gain de poids (g) | 460 ± 58,8 a | 496 ± 36,5 b | 495 ± 90,0 b | 516 ± 56,6 b | 510 ± 37,5 b | 493 ± 44,1 ab |
| Consommation (g) | 749 ± 82,9 a | 756 ± 50,3 a | 713 ± 118,8 ab | 722 ± 68,0 ab | 701 ± 51,9 bc | 665 ± 52,8 c |
| Indice de consommation (g/g) | 1,63 ± 0,063 a | 1,53 ± 0,048 b | 1,44 ± 0,067 c | 1,41 ± 0,060 d | 1,37 ± 0,043 de | 1,35 ± 0,042 e |

B M-S : Basal Maïs-Soja ; µA : microalgue

Les valeurs EMA et EMAn de la « microalgue, souche 4, culture 22h » (tableau 12) sont respectivement mesurées à 2785 kcal/kg MS et 2296 kcal/kg MS. Il est à noter que ces valeurs sont dans la gamme des référence moyenne *in vivo* de la mesure de l'EMAn tourteau de soja de classe protéique 46, 48 et 50 respectivement de 2303 ± 137, 2348 ± 248, 2365 ± 178 kcal/kg MS. Ainsi, la contribution de cette microalgue pour son apport en énergie est assimilable à celle d'un tourteau de soja de qualité standard.

**Tableau 12 (essai 14ALG069):**

| | Microalgue | | | Soybean meal | Soybean meal | Soybean meal |
|---|---|---|---|---|---|---|
| EMA, kcal/kg | 2635,0 | | | | | |
| EMA, kcal/kg MS | 2785,4 | | Classe protéique | 46 | 48 | 50 |
| EMAn, kcal/kg | 2172,0 | | N | n=15 | n=27 | n=80 |
| EMAn, kcal/kg MS | 2296,0 | | EMAn, kcal/kg MS | 2303 ±137 | 2348 ±248 | 2365 ±178 |
| A | | | B | | | |

A : Energie Métabolisable Apparente corrigée ou non du bilan azoté de la microalgue testée et mesurée chez le poulet de 23 jours d'âge.
B : Valeurs de références *in vivo* mesurées selon le même modèle et pour différentes classes protéiques de tourteaux de soja (références Adisseo, 2012).

**Exemple 2.7 : Tests de choix et mesure de consommation chez le poussin d'aliments comprenant la biomasse selon l'invention**

Dans chacun des essais présentés ci-dessous, la « microalgue, souche 4, culture 22h » comme les autres matières premières, a été broyée sur une grille de 3mm a été incorporée à des taux croissants de 5, 10, et 15% dans le régime maïs-soja, en ajustant la formulation de manière à avoir 4 régimes iso-énergétiques, iso-protéiques et iso-lysine (tableau 13), dont la composition est donnée dans le tableau 14.

**Tableau 13 (essai 14ALG069):** Coefficient de digestibilité apparente du phosphore de la microalgue testée et mesurée chez le poulet de 23 jours d'âge par régression linéaire

| | Microalgue |
|---|---|
| Phosphore digestible (%MS)[1] | 0,86 |
| CUDa phosphore (%)[2] | 35,7 |

[1] Intervalles de confiance : 0.576% - 1.143%
[2] Calculé selon la formule : CUDa = P digestible (%)/P total aliment (%)

**Tableau 14 (essai 14ALG081):** Formules des aliments expérimentaux utilisés dans les tests de consommation et de choix chez le poussin

| %, tel que fournit | Aliment contrôle | Aliment 5% microalgue | Aliment 10% microalgue | Aliment 15% microalgue |
|---|---|---|---|---|
| Maïs | 52,90 | 54,28 | 55,67 | 57,06 |
| Tourteau de soja 48% | 35,40 | 29,50 | 23,59 | 17,69 |
| Graine de soja extrudée | 4,00 | 4,00 | 4,00 | 4,00 |
| Huile de soja | 3,55 | 3,10 | 2,65 | 2,20 |
| Phosphate bicalcique | 1,92 | 1,78 | 1,63 | 1,49 |
| Carbonate de Calcium | 1 , 01 | 1,13 | 1 , 25 | 1,36 |
| Sel | 0,36 | 0,35 | 0,34 | 0,32 |
| DL-méthionine | 0,25 | 0,24 | 0,23 | 0,22 |
| L-Lysine HCl | 0,01 | 0,02 | 0,04 | 0,06 |
| Premix | 0,6 | 0,6 | 0,6 | 0,6 |
| Microalgue | 0 | 5 | 10 | 15 |

(suite)

| %, tel que fournit | Aliment contrôle | Aliment 5% microalgue | Aliment 10% microalgue | Aliment 15% microalgue |
|---|---|---|---|---|
| Protéine brute | 22 | 22 | 22 | 22 |
| Lysine | 1,25 | 1,25 | 1,25 | 1,25 |
| Méthionine | 0,58 | 0,59 | 0,60 | 0,61 |
| Met+Cys | 0,95 | 0,96 | 0,96 | 0,96 |
| Threonine | 0,86 | 0,87 | 0,88 | 0,88 |
| Dig Lysine | 1,12 | 1,12 | 1,12 | 1,12 |
| Dig Méthionine | 0,57 | 0,58 | 0,58 | 0,59 |
| Dig Met+Cys | 0,85 | 0,85 | 0,85 | 0,85 |
| Dig Threonine | 0,74 | 0,75 | 0,76 | 0,77 |
| Dig Tryptophane | 0,25 | 0,24 | 0,24 | 0,24 |
| Calcium | 0,95 | 0,95 | 0,95 | 0,95 |
| Phosphore total | 0,72 | 0,77 | 0,83 | 0,88 |
| Phosphore disponible | 0,40 | 0,40 | 0,40 | 0,40 |
| Energie Métabolisable, kcal/kg | 3000 | 3000 | 3000 | 3000 |

Deux essais expérimentaux ont été conduits en parallèle (figure 2). Dans le premier essai (test de choix), la consommation est mesurée lorsque l'animal a le choix entre un aliment supplémenté en microalgue et un aliment starter maïs-soja. Dans le second (mesure de consommation), la consommation est mesurée lorsque l'animal n'a accès qu'à un seul aliment, supplémenté ou non avec la microalgue.

Dans l'essai 1, les données de consommation à chaque heure de mesure au 7$^{ième}$ et 9$^{ième}$ jour d'âge ont été analysées selon une procédure de test apparié (XLSTAT 2010.4.02 © Addinsoft 1995-2010) considérant que la consommation d'une mangeoire est dépendante de la consommation de l'autre dans chaque cage.

Les données de consommation et de poids de l'essai 2 ont été analysées pour un effet aliment selon une procédure ANOVA (XLSTAT 2010.4.02 © Addinsoft 1995-2010) d'après le modèle suivant à 95% d'intervalle de confiance :

$$Y_i = \mu + a_i + b_i Y = \text{paramètre}$$

Avec

Y = paramètre
$\mu$ = moyenne
$a_i$ = effet de l'aliment
$b_i$ = effet du bloc

Pour chaque différence significative, les moyennes sont analysées par un pair-wise Fisher's LSD test.

**1) Essai 1** - **test de choix**

Pour l'essai avec choix de l'aliment, environ 200 poussins mâles (Ross PM3) de 1 jour ont été installés par groupe de 20 environ dans des cages métaboliques dédoublées et nourris avec un aliment de démarrage standard (starter) à base de blé, de maïs et de tourteau de soja. A 6 jours d'âge les poulets sont mis à jeun 2h avant d'être pesés et répartis par classes de poids. Cent-vingt poulets sont ainsi sélectionnés, installés par groupes de 4 dans 30 cages métaboliques dédoublées et assignés au jour 7 à l'un des traitements expérimentaux selon leur poids (10 répétitions par traitement). Chaque cage contient 2 mangeoires contenant des aliments différents, correspondant aux 3 traitements expérimentaux suivants :

- Traitement 1 : starter maïs-soja (mangeoire 1) et microalgue 5% (mangeoire 2)
- Traitement 2 : starter maïs-soja (mangeoire 1) et microalgue 10% (mangeoire 2)
- Traitement 3 : starter maïs-soja (mangeoire 1) et microalgue 15% (mangeoire 2)

Les aliments et l'eau sont distribués *ad libitum* tout au long de l'essai. Les aliments expérimentaux sont présentés en granulés de diamètre 3,2 mm. Une mesure des consommations est réalisée à T0 + 1h, T0 + 2h, T0 + 3 h, T0 + 4h, et T0 + 6h au 7ième et 9ième jour d'âge, avec inversion des mangeoires toutes les heures dans les cages. Entre les deux mesures de consommation (jour 8), les animaux reçoivent l'aliment démarrage blé-maïs-soja.

Les résultats démontrent (cf. figures 3-5) que les jeunes animaux n'ont pas présenté de rejet des aliments supplémentés en microalgue et ce, quel que soit le taux d'incorporation. A ces doses, la microalgue ne présente donc aucun problème d'appétence. Les jeunes poussins tendent même à préférer les aliments supplémentés à 5 et 10 % par la microalgue (figure 5) par rapport à l'aliment classique maïs-soja, ce qui s'inverse numériquement lorsque la microalgue est rajoutée à 15%.

## 2) Essai 2- mesure de consommation

Dans un deuxième test les poussins n'ont accès qu'à un seul type d'aliment, supplémenté ou non avec la microalgue (figure 6). Environ 400 poussins males (Ross PM3) de 1 jour ont été pesés et répartis par classe de poids. Deux-cent-quarante animaux sélectionnés sont placés par 4 au jour 1 dans 60 cages non dédoublées par bloc de poids homogène (15 blocs de 4 cages) avec une mangeoire par cage, chacune avec un aliment expérimental (15 répétitions par aliment). Dans chaque cage, les 4 poussins sont identifiés individuellement. Les aliments et l'eau sont distribués *ad libitum* tout au long de l'essai. Les aliments expérimentaux sont l'aliment de démarrage (starter) maïs-soja supplémenté avec 0, 5, 10 ou 15% de microalgue, également utilisés dans l'essai 1 avec choix de l'aliment. Les poids vifs individuels sont mesurés à 1, 7 et 9 jours d'âge. Les refus sont pesés et les consommations par cage mesurées à 7 et 9 jours d'âge. De 0 à 7 jours d'âge, les animaux nourris avec l'aliment contenant 10 % de microalgue ont un gain de poids significativement amélioré par rapport au contrôle et aux deux autres régimes supplémentés. L'indice de consommation mesuré pour le régime supplémenté à 10% est significativement amélioré par rapport au contrôle et le régime supplémenté à 15% (1,089 vs 1,156). Par ailleurs, la supplémentation en microalgue n'a pas d'effet sur la consommation d'aliment quand on la compare au contrôle maïs-soja (122,6, 125,7, 135,6, 124,6 g respectivement pour le contrôle et les 3 régimes supplémentés de 5 à 15%).

Sur la période totale de mesure de 0 à 9 jours d'âge (figure 7), l'effet positif de l'aliment supplémenté avec 10% de microalgue sur le gain de poids et l'indice de consommation se confirme.

Les résultats de consommation (en condition de non choix) montrent que la « microalgue, souche 4, culture 22h » testée peut être incluse jusqu'à 15% dans des aliments équilibrés à base de maïs-soja sans affecter les performances des poussins de 0 à 9 jours d'âge.

## REFERENCES

- Becker E.W. (2007) Biotechnol. Adv., 25: 207-210Henman *et al.,* 2012
- Bourdillon A., Carré B., Conan L., Francesch M., Fuentes M., Huyghebaert G., Janssen W.M., Leclercq B., Lessire M., McNab J., Rigoni M. et Wiseman J., 1990b. Br.Poult.Sci., (31), 567-576.
- Carré et Brillouet (1989, Détermination of water insoluble cell walls in feeds: interlaboratory study. Journal Association of Official Analytical Chemists, 72, 463-467).
- Green S., Bertrand S., Duron M., Maillard R., 1987. Br. Poult. Sci. (28), 631-641.
- Green S., Bertrand S., Duron M., Maillard R., 1987. Br. Poult. Sci. (28), 631-641.
- Henman D.J. (2012) Report 4A-102, CRC Australia, 15 p
- Hill F.W. et Anderson D.L., 1958. J.Nutr., (64), 587-603.
- Lieve M. L. Laurens, Thomas A. Dempster, Howland D. T. Jones, Edward, J. Wolfrum, Stefanie Van Wychen, Jordan S. P. McAllister, Michelle Rencenberger, Kylea J. Parchert, and Lindsey M. Gloe. Algal Biomass Constituent Analysis: Method Uncertainties and Investigation of the Underlying Measuring Chemistries. Anal. Chem. 2012, 84, 1879-1887Bourdillon *et al.,* 1990
- Prosky L., Asp N.-G., Schweitzer T.F., DeVries J.W., Furda I., 1988. Détermination of total dietary fiber in foods and food products: interlaboratory study. Journal of the Association of Official Analytical Chemists, 71:1017-1023.
- Skrede A., Mydland L.T., Ahlstrøm Ø., Reitan K.I.,. Gislerød H.R., Øverland M. (2011) J. Anim. Feed Sci., 20: 131-14211
- Terpstra K., De Hart N., 1974. J. Anim. Physiol.Anim. Nutr., (32), 306-320.

- WO 2010/051489
- WO 97/37032

**Revendications**

1. Biomasse de Thraustochytrides non-génétiquement modifiées choisis parmi les genres *Aurantiochytrium* et *Schizochytrium,* dont une quantité substantiellement majoritaire de Thraustochytrides n'est pas dégradée, **caractérisée en ce qu'**elle comprend, en poids par rapport au poids de la matière sèche, au moins 35 % de protéines et moins de 20% de matière grasse et **en ce qu'**elle n'a pas subi de traitements après récolte qui modifient sa composition en acides aminés et en matière grasse.

2. Biomasse selon la revendication 1, **caractérisée en ce qu'**elle comprend au moins 45 % de protéines.

3. Biomasse selon la revendication 1, **caractérisée en ce qu'**elle comprend au moins 60 % de protéines.

4. Biomasse selon l'une des revendications 1 à 3, **caractérisée en ce qu'**elle comprend moins de 10% de matière grasse.

5. Biomasse selon l'une des revendications 1 à 4, **caractérisée en ce que** lesdits Thraustochytrides sont choisis parmi les espèces *Aurantiochytrium mangrovei* CCAP 4062/2 ; *Aurantiochytrium mangrovei* CCAP 4062/3 ; *Aurantiochytrium mangrovei* CCAP 4062/4 ; *Aurantiochytrium mangrovei,* CCAP 4062/5 ; *Aurantiochytrium mangrovei* CCAP 4062/6 ; *Aurantiochytrium mangrovei* CCAP 4062/1 *; Schizochytrium sp.* 4087/3 ; *Schizochytrium sp.* CCAP 4087/1 ; *Schizochytrium sp.* CCAP 4087/4 ; *Schizochytrium sp.* CCAP 4087/5.

6. Biomasse selon l'une quelconque des revendications 1 à 5, **caractérisée en ce qu'**elle présente un taux d'humidité de 1% à 95 %.

7. Biomasse selon la revendication 6, **caractérisée en ce qu'**elle présente un taux d'humidité de 70% à 90 %.

8. Biomasse selon la revendication 6, **caractérisée en ce qu'**elle présente un taux d'humidité de 1% à 10%.

9. Procédé de production d'une biomasse telle que définie dans l'une des revendications 1 à 8, **caractérisé en ce qu'**il comprend

a. Une première étape de culture des Thraustochytrides dans un milieu de culture défini comprenant une source de carbone, une source d'azote, une source de phosphore et des sels, ledit milieu défini ne comportant pas de matières organiques riches ou complexes et ladite première étape a) étant divisée en 2 sous-étapes, une première sous-étape

a1) de croissance dans le milieu de culture approprié jusqu'à obtenir des teneurs en source de carbone dans le milieu inférieure à 20 g/L suivie d'une deuxième sous-étape
a2) de production dans laquelle on ajoute au milieu de culture, simultanément ou successivement, une ou plusieurs solution(s) d'enrichissement en source de carbone pour maintenir la teneur en source de carbone entre 0 et 50 g/L, en source d'azote pour maintenir la teneur en azote entre 0,5 et 5 g/L et/ou en source de phosphore pour maintenir la teneur en phosphore entre 0,5 et 5 g/L de manière à maintenir dans le milieu de culture des teneurs en azote et en phosphore non limitantes pour la croissance jusqu'à l'obtention d'une densité de culture d'au moins 40 g/L en matière sèche ;

b. une deuxième étape de récupération de la biomasse obtenue à la première étape par séparation de ladite biomasse du milieu de culture.

10. Procédé selon la revendication 9, **caractérisé en ce qu'**il comprend une troisième étape :
c. de séchage de la biomasse récupérée à la deuxième étape.

11. Utilisation d'une biomasse de Thraustochytrides selon l'une quelconque des revendications 1 à 8 pour l'amélioration des performances des animaux.

**12.** Utilisation d'une biomasse de Thraustochytrides selon l'une quelconque des revendications 1 à 8 dans l'alimentation humaine ou animale.

**Patentansprüche**

**1.** Biomasse von nicht genetisch modifizierten Thraustochytriden, die aus den Gattungen *Aurantiochytrium* und *Schizochytrium* ausgewählt sind, in der eine im Wesentlichen überwiegende Menge von Thraustochytriden nicht abgebaut wird, **dadurch gekennzeichnet, dass** sie, bezogen auf das Gewicht relativ zu dem Gewicht der Trockenmasse, mindestens 35 % Proteine und mindestens 20 % Fett umfasst und dass sie nach der Ernte keinen Behandlungen unterzogen wird, die ihre Zusammensetzung von Aminosäuren und Fett modifiziere.

**2.** Biomasse nach Anspruch 1, **dadurch gekennzeichnet, dass** sie mindestens 45 % Proteine umfasst.

**3.** Biomasse nach Anspruch 1, **dadurch gekennzeichnet, dass** sie mindestens 60 % Proteine umfasst.

**4.** Biomasse nach einem der Ansprüche 1 bis 3, **dadurch gekennzeichnet, dass** sie weniger als 10 % Fett umfasst.

**5.** Biomasse nach einem der Ansprüche 1 bis 4, **dadurch gekennzeichnet, dass** die Thraustochytriden aus den Spezies *Aurantiochytrium magrovei* CCAP 406212; *Aurantiochytrium magrovei* CCAP 4062/3; *Aurantiochytrium magrovei* CCAP 4062/4; *Aurantiochytrium magrovei* CCAP 4062/5; *Aurantiochytrium magrovei* CCAP 4062/6; *Aurantiochytrium magrovei* CCAP 4062/1; *Schizochytrium sp.* 4087/3; *Schizochytrium sp.* CCAP 4087/1; *Schizochytrium sp.* CCAP 4087/4; *Schizochytrium sp.* CCAP 4087/5 ausgewählt sind.

**6.** Biomasse nach einem der Ansprüche 1 bis 5, **dadurch gekennzeichnet, dass** sie einen Feuchtigkeitsgehalt von 1 % bis 95 % aufweist.

**7.** Biomasse nach Anspruch 6, **dadurch gekennzeichnet, dass** sie einen Feuchtigkeitsgehalt von 70 % bis 90 % aufweist.

**8.** Biomasse nach Anspruch 6, **dadurch gekennzeichnet, dass** sie einen Feuchtigkeitsgehalt von 1 % bis 10 % aufweist.

**9.** Verfahren zur Produktion einer wie in einem der Ansprüche 1 bis 8 definierten Biomasse, **dadurch gekennzeichnet, dass** es umfasst

a. Einen ersten Schritt eines Kultivierens von Thraustochytriden in einem definierten Kulturmedium, umfassend eine Quelle von Kohlenstoff, eine Quelle von Stickstoff, eine Quelle von Phosphor und Salzen, wobei das definierte Medium keine reichhaltigen oder komplexen organischen Substanzen umfasst und der erste Schritt a) in 2 Teilschritte aufgeteilt ist, einen ersten Teilschritt

a1) eines Wachsenlassens in einem adäquaten Kulturmedium, bis Gehalte der Quelle von Kohlenstoff in dem Medium von weniger als 20 g/L erhalten werden, gefolgt von einem zweiten Teilschritt
a2) eines Produzierens, in dem gleichzeitig oder nacheinander eine oder mehrere Lösungen zur Anreicherung der Quelle von Kohlenstoff, um einen Gehalt der Quelle von Kohlenstoff zwischen 0 und 50 g/L aufrechtzuerhalten, der Quelle von Stickstoff, um einen Gehalt von Stickstoff zwischen 0,5 und 50 g/L aufrechtzuerhalten, und/oder der Quelle von Phosphor, um einen Gehalt von Phosphor zwischen 0,5 und 50 g/L aufrechtzuerhalten, dem Kulturmedium zugegeben werden, um die nicht einschränkenden Gehalte von Stickstoff und Phosphor in dem Kulturmedium für das Wachstum aufrechtzuerhalten, bis zum Erhalt einer Kulturdichte von mindestens 40 g/L Trockenmasse;

b. einen zweiten Schritt eines Gewinnens der in dem ersten Schritt erhaltenen Biomasse durch Trennung der Biomasse von dem Kulturmedium.

**10.** Verfahren nach Anspruch 9, **dadurch gekennzeichnet, dass** es einen dritten Schritt umfasst:
c. Eines Trocknens der in dem zweiten Schritt gewonnenen Biomasse.

**11.** Verwendung einer Biomasse von Thraustochytriden nach einem der Ansprüche 1 bis 8 zur Verbesserung der Leistungen von Tieren.

**12.** Verwendung einer Biomasse von Thraustochytriden nach einem der Ansprüche 1 bis 8 in der Ernährung von Menschen oder Tieren.

**Claims**

**1.** Biomass of Thraustochytrids non-genetically modified selected among the genus *Aurantiochytrium* and *Schyzochytrium,* of which a substantially major amount of Thraustochytrid is not degraded, **characterized in that** it comprises, by weight relative to the weight of dry matter, at least 35% protein and less than 20% fat, and **in that** it has not undergone treatments after harvesting that modify its amino acid and fat composition.

**2.** Biomass according to claim 1, **characterized in that** it comprises at least 45% protein.

**3.** Biomass according to claim 1, **characterized in that** it comprises at least 60% protein.

**4.** Biomass according to one of claims 1 to 3, **characterized in that** it comprises less than 10% fat.

**5.** Biomass according to one of claims 1 to 4, **characterized in that** the said Thraustochytrids are chosen from the species *Aurantiochytrium mangrovei* CCAP 4062/2; *Aurantiochytrium mangrovei* CCAP 4062/3; *Aurantiochytrium mangrovei* CCAP 4062/4; *Aurantiochytrium mangrovei,* CCAP 4062/5; *Aurantiochytrium mangrovei* CCAP 4062/6; *Aurantiochytrium mangrovei* CCAP 4062/1; *Schizochytrium sp.* 4087/3; *Schizochytrium sp.* CCAP 4087/1; *Schizochytrium sp.* CCAP 4087/4; *Schizochytrium sp.* CCAP 4087/5.

**6.** Biomass according to any one of claims 1 to 5, **characterized in that** it has a moisture content of 1% to 95%.

**7.** Biomass according to claim 6, **characterized in that** it has a moisture content of 70% to 90%.

**8.** Biomass according to claim 6, **characterized in that** it has a moisture content of 1% to 10%.

**9.** Process for producing a biomass as defined in one of claims 1 to 8, **characterized in that** it comprises

a. A first step of culturing Thraustochytrids in a defined culture medium comprising a carbon source, a nitrogen source, a phosphorus source and salts, said defined medium not comprising rich or complex organic matter and said first step a) being divided into 2 substeps, a first substep

a1) of growth in the appropriate culture medium until obtaining in the medium contents in carbon source of less than 20 g/L followed by a second sub-step
a2) of production in which one adds to the culture medium, simultaneously or successively, one or more enrichment solutions in carbon source to maintain carbon source content between 5 and 50 g/L, in nitrogen source to maintain the nitrogen content between 0,5 and 5 g/L and/or in phosphorus source to maintain the phosphorus content between 0,5 and 5 g/L, so as to maintain in the culture medium nitrogen and phosphorus contents that do not limit growth until the obtention of a culture density of at least 40 g/L in dry matter;

b. a second step of recovering the biomass obtained in the first step by separating said biomass from the culture medium.

**10.** Process according to claim 9, **characterized in that** it comprises a third step : c. drying the biomass recovered in the second step.

**11.** Use of a Thraustochytrids biomass according to any one of claims 1 to 8 for the improvement of animal performance.

**12.** Use of a Thraustochytrids biomass according to any one of claims 1 to 8 for human or animal feed.

Figure 1

**200 poussins mâles**

**120 poulets mis en lots (10 répétitions) en aléatoire**

| Jour 0 | Jour 5 | | Jour 7 | Jour 8 | Jour 9 | Jour 9 |
|---|---|---|---|---|---|---|
| Groupes de 20 en cages doubles Starter granulé blé-maïs-soja | Jeûne 2h | 4 poussins/cage dédoublée Starter granulés courts | **Choix aliments expérimentaux 6h** | Starter granulé blé-maïs-soja | **Choix aliments expérimentaux 6h** | |

Contrôles consommation toutes les 2h

Contrôles consommation toutes les 2h

*Inversion des 2 mangeoires toutes les heures*

*Inversion des 2 mangeoires toutes les heures*

**400 poussins mâles**

**240 poulets mis en lots (15 répétitions) en aléatoire**

| Jour 0 | Jour 6 | Jour 7 | | Jour 9 |
|---|---|---|---|---|
| **4 poussins/cage non dédoublée Aliments expérimentaux** | Jeûne 2h | **4 poussins/cage non dédoublée Aliments expérimentaux** | | Jeûne 17 h |

Pesée individuelle

Pesée refus aliment

Pesée par cage

Contrôles de la consommation

Pesée par cage

**Figure 2**

EP 3 325 605 B1

Figure 3

Figure 4

Figure 5

* $P \leq 0{,}1$
** $P \leq 0{,}05$

Figure 6

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010051489 A
- WO 9737032 A
- WO 2015004402 A
- WO 2012175027 A

**Littérature non-brevet citée dans la description**

- **FOLCH J.** A simple method for the isolation and purification of total lipids from animal tissues. *J Biol Chem.,* Mai 1957, vol. 226 (1), 497-509
- **YOKOYAMA.** Taxonomic rearrangement of the genus Schizochytrium sensu lato based on morphology, chemotaxonomic characteristics, and 18S rRNA gene phylogeny (Thraustochytriaceae, Labyrinthulomycetes): emendation for Schizochytrium and érection of Aurantiochytrium and Oblongichytrium gen. nov. *Mycoscience,* 2007, vol. 48, 199-211
- **BECKER E.W.** *Biotechnol. Adv.,* 2007, vol. 25, 207-210
- **BOURDILLON A. ; CARRÉ B. ; CONAN L. ; FRANCESCH M. ; FUENTES M. ; HUYGHEBAERT G. ; JANSSEN W.M. ; LECLERCQ B. ; LESSIRE M. ; MCNAB J.** *Br.Poult.Sci.,* 1990, vol. 31, 567-576
- **CARRÉ ; BRILLOUET.** Détermination of water insoluble cell walls in feeds: interlaboratory study. *Journal Association of Official Analytical Chemists,* 1989, vol. 72, 463-467
- **GREEN S. ; BERTRAND S. ; DURON M. ; MAILLARD R.** *Br. Poult. Sci.,* 1987, vol. 28, 631-641
- **HENMAN D.J.** Report 4A-102. CRC, 2012, 15
- **HILL F.W. ; ANDERSON D.L.** *J.Nutr.,* 1958, vol. 64, 587-603
- **LIEVE M. L. LAURENS ; THOMAS A. DEMPSTER ; HOWLAND D. T. JONES ; EDWARD, J. WOLFRUM ; STEFANIE VAN WYCHEN ; JORDAN S. P. MCALLISTER ; MICHELLE RENCENBERGER ; KYLEA J. PARCHERT ; LINDSEY M. GLOE.** Algal Biomass Constituent Analysis: Method Uncertainties and Investigation of the Underlying Measuring Chemistries. *Anal. Chem.,* 2012, vol. 84, 1879
- **PROSKY L. ; ASP N.-G. ; SCHWEITZER T.F. ; DEVRIES J.W. ; FURDA I.** Détermination of total dietary fiber in foods and food products: interlaboratory study. *Journal of the Association of Official Analytical Chemists,* 1988, vol. 71, 1017-1023
- **SKREDE A. ; MYDLAND L.T. ; AHLSTRØM Ø. ; REITAN K.I. ; GISLERØD H.R. ; ØVERLAND M.** *J. Anim. Feed Sci.,* 2011, vol. 20, 131-14211
- **TERPSTRA K. ; DE HART N.** *J. Anim. Physiol.Anim. Nutr.,* 1974, vol. 32, 306-320